# EUROPEAN PATENT APPLICATION

(11) **EP 4 403 581 A1**
(43) Date of publication of application: **24.07.2024**
(21) Application number: 23780828.2
(22) Date of filing: 30.03.2023
(51) Int. Cl.: C07K 16/40, C12Q 1/6886, G01N 33/53, G01N 33/574

(54) **ANTI-CK2 ALPHA ANTIBODY OR FRAGMENT THEREOF**

(30) Priority: 30.03.2022 JP 2022055606
(71) Applicant: Homma, Miwako, Fukushima-shi, Fukushima 960-1295 (JP)
(72) Inventor: Homma, Miwako, Fukushima-shi, Fukushima 960-1295 (JP)
(74) Representative: Mewburn Ellis LLP
(86) International application number: PCT/JP2023/013069
(87) International publication number: WO 2023/190820

(57) **Abstract**

An object of the present invention is to provide a new anti-CK2α antibody improved in specificity and sensitivity. Provided is an anti-CK2α antibody or a fragment thereof, including: (1) a heavy chain variable region comprising CDR1 consisting of the amino acid sequence of SEQ ID NO: 5, CDR2 consisting of the amino acid sequence of SEQ ID NO: 6, and CDR3 consisting of an amino acid sequence FV, and a light chain variable region comprising: CDR1 consisting of the amino acid sequence of SEQ ID NO: 8, CDR2 consisting of the amino acid sequence of SEQ ID NO: 9, and CDR3 consisting of the amino acid sequence of SEQ ID NO: 10; (2) a heavy chain variable region comprising: CDR1 consisting of the amino acid sequence of SEQ ID NO: 17, CDR2 consisting of the amino acid sequence of SEQ ID NO: 18, and CDR3 consisting of an amino acid sequence FV, and a light chain variable region comprising: CDR1 consisting of the amino acid sequence of SEQ ID NO: 20, CDR2 consisting of the amino acid sequence of SEQ ID NO: 21, and CDR3 consisting of the amino acid sequence of SEQ ID NO: 22.

## Description

### Technical Field

The present invention relates to an anti-CK2α antibody or a fragment thereof, a kit for predicting the prognosis of a cancer patient, and a method for predicting the prognosis of a cancer patient.

### Background Art

In Japan, cancer is the most frequent cause of death among all causes of death, accounting for about 30% of all deaths. For example, breast cancer is the primary cause of death among women of 30 to 64 years old, and in 2018 the number of deaths due to breast cancer was about 14,000. Although advances in cancer detection and/or treatment methods have improved the survival rate of cancer patients, there are still some patients with poor prognosis having high risk of recurrence (relapse), metastasis, or death. Therefore, in order to improve the quality of treatment of cancer including breast cancer, it is very important to predict the prognosis of a cancer patient, and to individually manage the cancer patient according to the result.

The present inventor found that CK2α (Casein kinase 2α) protein is a new biomarker that can highly precisely predict the prognosis of a cancer patient including a breast cancer patient (Patent Literature 1). The CK2α protein is present throughout the cell in normal cells, but in cancer cells, the protein is highly expressed in the nucleus, and localized to the nucleolus in association with poor prognosis. The present inventors revealed that the intranuclear expression level and nucleolus staining level of the CK2α protein show strong correlation with the poor prognosis of a cancer patient, and are strongly associated also with recurrence risk (Patent Literature 1).

The method of using the CK2α protein in the nucleolus as a biomarker is a break-through technology that enables the prognosis of a cancer patient to be predicted with very high precision. However, existing anti-CK2α antibodies for use in the prediction of prognosis do not have sufficient specificity or sensitivity to the CK2α protein.

Accordingly, a new anti-CK2α antibody that is improved in specificity and sensitivity compared with conventional anti-CK2α antibodies is necessary.

### Citation List

### Patent Literature

Patent Literature 1: WO2021/132544

### Summary of Invention

### Technical Problem

An object of the present invention is to provide a new anti-CK2α antibody improved in specificity and sensitivity.

### Solution to Problem

The CK2α protein constitutes CK2 (Casein kinase 2) that is serine/threonine kinase, together with the CK2α' protein and the CK2β protein. The structure of the CK2α protein is similar to that of the CK2α' protein, and the two proteins could not be distinguished by conventional anti-CK2α antibodies.

To solve the above-described problem, the present inventor has developed mAb (6A3) as a new antibody that exhibits very high specificity to the CK2α protein. Most of anti-CK2α antibodies produced by mice immunized with the human CK2α protein exhibit cross-reactivity to the CK2α' protein. The present inventor applied the criteria of binding only to the CK2α protein, but not binding to the CK2α' protein, and thereby succeeded in developing mAb (6A3) having very high specificity to the CK2α protein. In Western blotting and immunoprecipitation for the CK2α protein, mAb (6A3) exhibited remarkably high specificity and sensitivity, compared with a conventional antibody. Furthermore, mAb (6A3) detects the CK2α protein localized to the nucleolus in cancer tissues derived from a cancer patient with poor prognosis with very high specificity and sensitivity, and provides an very useful tool in the biomarker detection. The present invention is based on the above-described findings, and provides the following.

(1) An anti-CK2α antibody or a fragment thereof, comprising:
   (i) a heavy chain variable region comprising:
      CDR1 consisting of the amino acid sequence of SEQ ID NO: 5,
      CDR2 consisting of the amino acid sequence of SEQ ID NO: 6, and
      CDR3 consisting of an amino acid sequence FV, and
      a light chain variable region comprising:
      CDR1 consisting of the amino acid sequence of SEQ ID NO: 8,
      CDR2 consisting of the amino acid sequence of SEQ ID NO: 9, and
      CDR3 consisting of the amino acid sequence of SEQ ID NO: 10;
   (ii) a heavy chain variable region comprising:
      CDR1 consisting of the amino acid sequence of SEQ ID NO: 17,
      CDR2 consisting of the amino acid sequence of SEQ ID NO: 18, and
      CDR3 consisting of an amino acid sequence FV, and
      a light chain variable region comprising:
      CDR1 consisting of the amino acid sequence of SEQ ID NO: 20,
      CDR2 consisting of the amino acid sequence of SEQ ID NO: 21, and
      CDR3 consisting of the amino acid sequence of SEQ ID NO: 22;
   (iii) a heavy chain variable region comprising:
      CDR1 consisting of the amino acid sequence of SEQ ID NO: 25,
      CDR2 consisting of the amino acid sequence of SEQ ID NO: 26, and
      CDR3 consisting of an amino acid sequence FV, and
      a light chain variable region comprising:
      CDR1 consisting of the amino acid sequence of SEQ ID NO: 28,
      CDR2 consisting of the amino acid sequence of SEQ ID NO: 29, and
      CDR3 consisting of the amino acid sequence of SEQ ID NO: 30;
   (iv) a heavy chain variable region comprising:
      CDR1 consisting of the amino acid sequence of SEQ ID NO: 33,
      CDR2 consisting of the amino acid sequence of SEQ ID NO: 34, and
      CDR3 consisting of an amino acid sequence FV, and
      a light chain variable region comprising:
      CDR1 consisting of the amino acid sequence of SEQ ID NO: 36,
      CDR2 consisting of the amino acid sequence of SEQ ID NO: 37, and
      CDR3 consisting of the amino acid sequence of SEQ ID NO: 38;
   (v) a heavy chain variable region comprising:
      CDR1 consisting of the amino acid sequence of SEQ ID NO: 41,
      CDR2 consisting of the amino acid sequence of SEQ ID NO: 42, and
      CDR3 consisting of the amino acid sequence of SEQ ID NO: 43, and
      a light chain variable region comprising:
      CDR1 consisting of the amino acid sequence of SEQ ID NO: 44,
      CDR2 consisting of the amino acid sequence of SEQ ID NO: 45, and
      CDR3 consisting of the amino acid sequence of SEQ ID NO: 46; or
   (vi) a heavy chain variable region comprising:
      CDR1 consisting of the amino acid sequence of SEQ ID NO: 49,
      CDR2 consisting of the amino acid sequence of SEQ ID NO: 50, and
      CDR3 consisting of the amino acid sequence of SEQ ID NO: 51, and
      a light chain variable region comprising:
      CDR1 consisting of the amino acid sequence of SEQ ID NO: 52,
      CDR2 consisting of the amino acid sequence of SEQ ID NO: 53, and
      CDR3 consisting of the amino acid sequence of SEQ ID NO: 54.
(2) The anti-CK2α antibody or a fragment thereof according to (1), comprising:
   (a) a heavy chain variable region consisting of the amino acid sequence of SEQ ID NO: 11, and a light chain variable region consisting of the amino acid sequence of SEQ ID NO: 12;
   (b) a heavy chain variable region consisting of the amino acid sequence of SEQ ID NO: 15, and a light chain variable region consisting of the amino acid sequence of SEQ ID NO: 16;
   (c) a heavy chain variable region consisting of the amino acid sequence of SEQ ID NO: 23, and a light chain variable region consisting of the amino acid sequence of SEQ ID NO: 24;
   (d) a heavy chain variable region consisting of the amino acid sequence of SEQ ID NO: 31, and a light chain variable region consisting of the amino acid sequence of SEQ ID NO: 32;
   (e) a heavy chain variable region consisting of the amino acid sequence of SEQ ID NO: 39, and a light chain variable region consisting of the amino acid sequence of SEQ ID NO: 40; or
   (f) a heavy chain variable region consisting of the amino acid sequence of SEQ ID NO: 47, and a light chain variable region consisting of the amino acid sequence of SEQ ID NO: 48.
(3) A kit for predicting the prognosis of a cancer patient, comprising the anti-CK2α antibody or a fragment thereof according to (1) or (2).
(4) The kit according to (3), wherein the prognosis comprises recurrence risk.
(5) The kit according to (3) or (4), wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.
(6) A method for predicting the prognosis of a cancer patient, the method comprising:
   a step of detecting a CK2α protein or a fragment thereof in a nucleolus in a cancer cell or a tissue obtained from a cancer patient; and
   a step of predicting a poor prognosis when a CK2α protein or a fragment thereof is detected in a nucleolus at a higher level compared with other cell fractions, and/or predicting a good prognosis when a CK2α protein or a fragment thereof is not detected in a nucleolus at a higher level compared with other cell fractions,
   wherein the CK2α protein or a fragment thereof is detected with the anti-CK2α antibody or a fragment thereof according to (1) or (2).
(7) The method according to (6), wherein the prognosis comprises recurrence risk.
(8) The method according to (6) or (7), wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.
(9) The method according to (8), wherein said cancer is breast cancer and whether a CK2α protein or a fragment thereof is detected or not is combined with at least one of classification by stage, classification by hormone receptor expression status, and classification by HER2 gene and/or protein expression status to predict the prognosis of a breast cancer patient.

The present specification encompasses the disclosure of Japanese Patent Application No. 2022-055606, to which the priority of the present application is claimed.

### Advantageous Effects of Invention

The present invention provides a new anti-CK2α antibody improved in specificity and sensitivity.

### Brief Description of Drawings

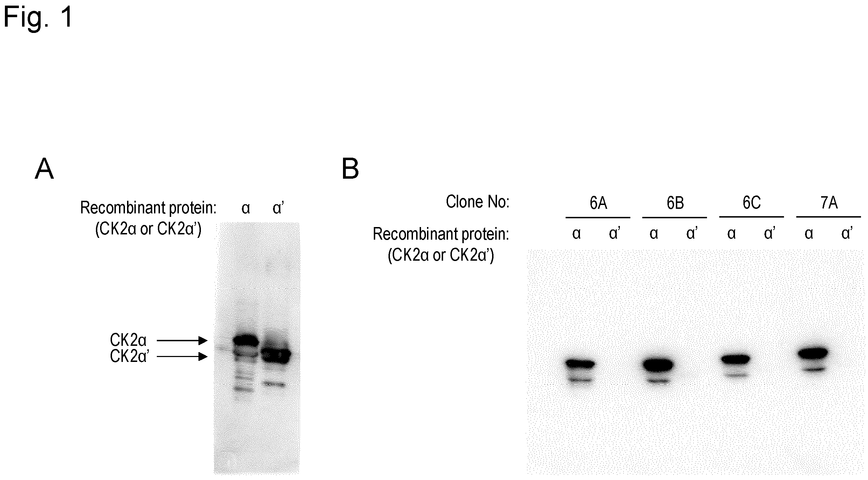

[Figure 1] Figure 1 shows the results of Western blotting performed with a mouse antiserum and a hybridoma culture supernatant. Figure 1A shows the results of Western blotting for the recombinant CK2α protein (α) and the recombinant CK2α' protein (α') using the antiserum of a mouse immunized with an antigen polypeptide. Figure 1B shows the results of Western blotting for the recombinant CK2α protein (α) and the recombinant CK2α protein (α') using the culture supernatants of clones 6A, 6B, 6C, and 7A.

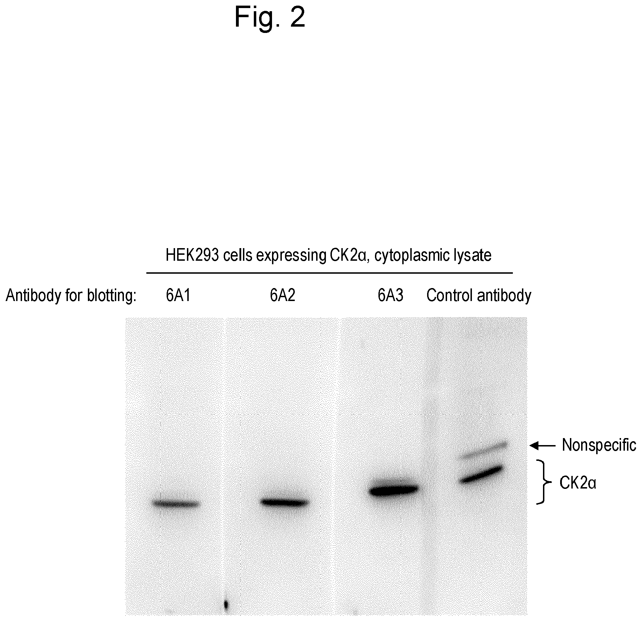

[Figure 2] Figure 2 shows the results of detection of the CK2α protein in a cytoplasmic lysate of HEK293 cells by Western blotting using the culture supernatants of clones 6A1, 6A2, and 6A3 and a commercially available mouse anti-CK2α monoclonal antibody (ab70774; Abcam, UK; shown as "Control antibody" in the figure). A nonspecific band indicated by the arrow was detected by the control antibody.

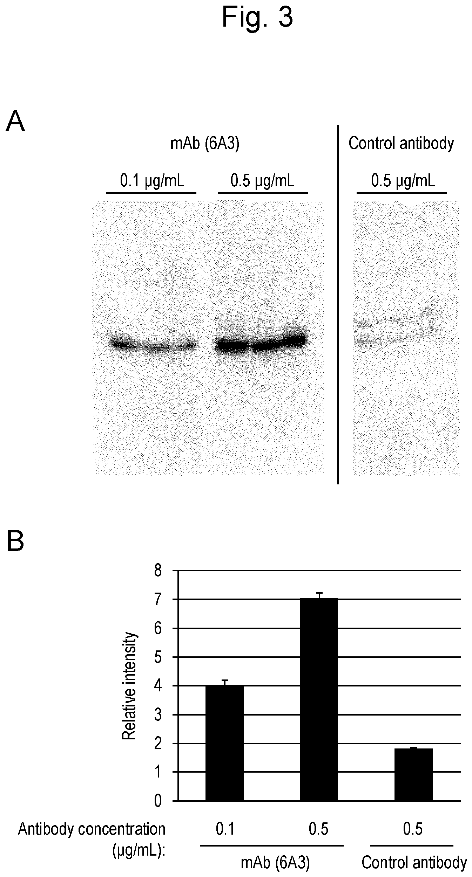

[Figure 3] Figure 3 shows the results of detection of the cellular endogenous CK2α protein in HEK293 cells by Western blotting using mAb (6A3) and a control antibody. Figure 3A shows the results of Western blotting. Figure 3B shows the results of quantifying the staining intensity of the band corresponding to the CK2α protein. The figures show the average of n = 3 results in each condition. Error bars indicate SEM.

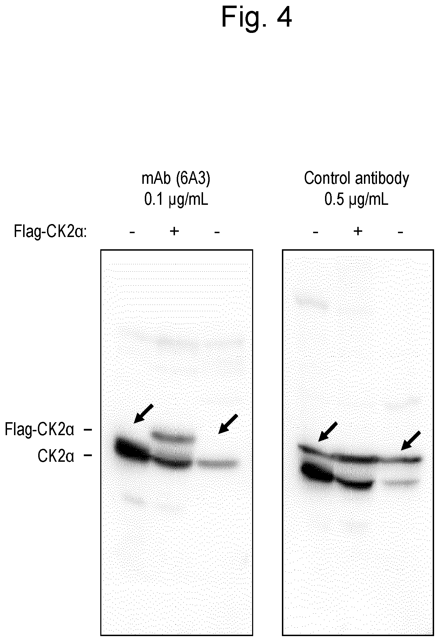

[Figure 4] Figure 4 shows the results of detection of the Flag-CK2α protein and the endogenous CK2α protein in a cytoplasmic lysate of HEK293 cells expressing the Flag-CK2α protein (the lane of Flag-CK2α (+)) and HEK293 cells not expressing the Flag-CK2α protein (the lane of Flag-CK2α (-)) by Western blotting using mAb (6A3) and a control antibody. Nonspecific bands were detected by the control antibody (shown by the arrows in the right panel), but not by mAb (6A3) (as shown by the arrows in the left panel).

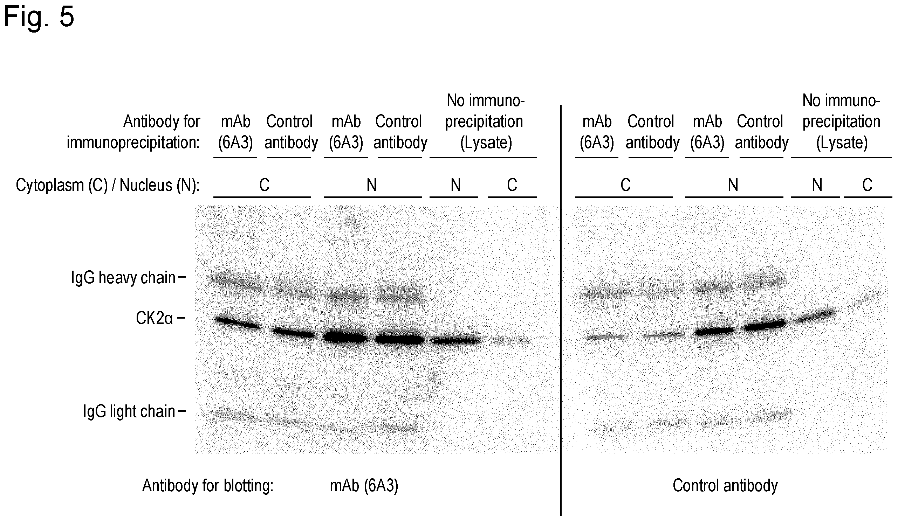

[Figure 5] Figure 5 shows the results of Western blotting for immunoprecipitates. The figure shows the results of Western blotting performed with mAb (6A3) or a control antibody for immunoprecipitates obtained from a lysate prepared from each of the cytoplasm (C) and the nucleus (N) of HEK293 cells by immunoprecipitation with mAb (6A3) or the control antibody. "No immunoprecipitation" means that the sample was loaded as a lysate of each of the cytoplasm (C) and the nucleus (N) for which immunoprecipitation was not performed.

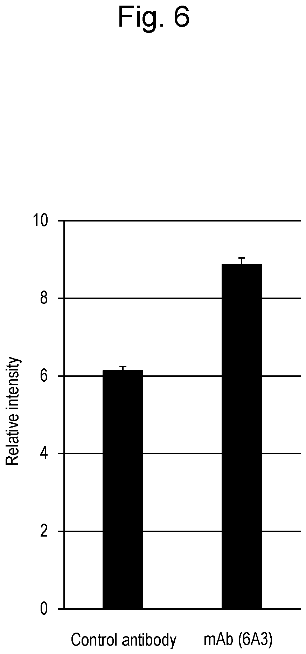

[Figure 6] Figure 6 shows the results of Western blotting performed for immunoprecipitates. Shown are the results of quantifying the staining intensities of the bands corresponding to the CK2α in the membranes on the left and right in Figure 5. The figure shows the average of n = 3 results, and error bars indicate SEM.

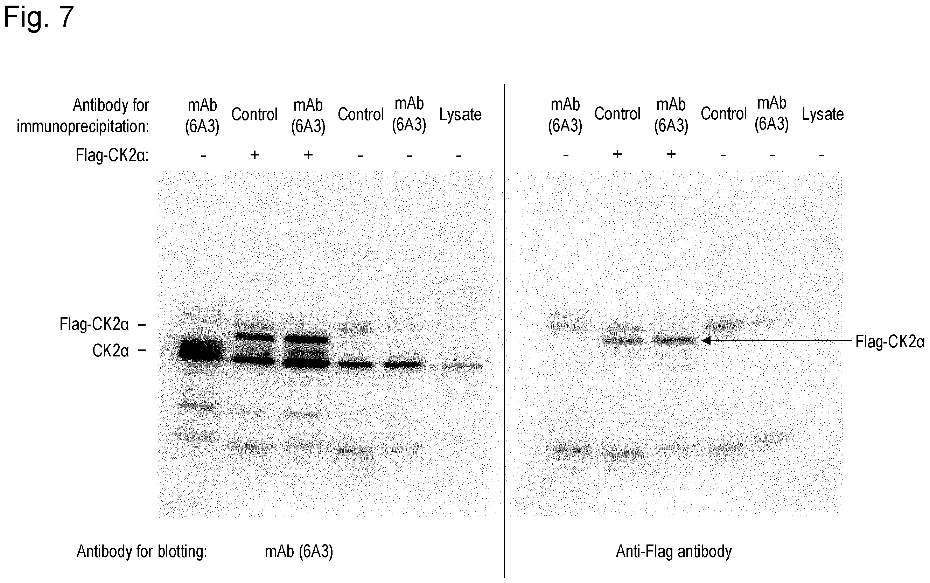

[Figure 7] Figure 7 shows the results of Western blotting for immunoprecipitates. The figure shows the results of Western blotting performed with mAb (6A3) or an anti-Flag antibody for immunoprecipitates which were obtained from a lysate of Flag-CK2α-expressing RPE cells by immunoprecipitation with mAb (6A3) or the control antibody.

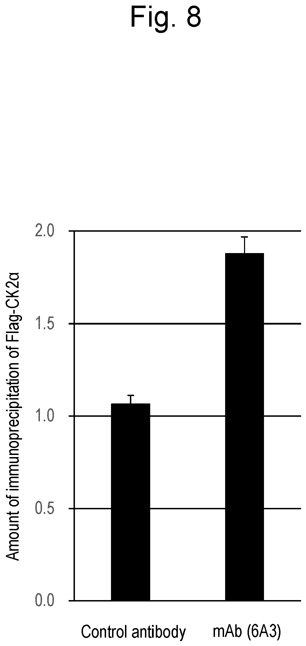

[Figure 8] Figure 8 shows the results of Western blotting for immunoprecipitates. The figure shows the results of quantifying the staining intensities of the bands (the two bands shown by the arrow in Figure 7) when mAb (6A3) or a control antibody was used as an antibody for immunoprecipitation in Figure 7. The figure shows the average of n = 3 results, and error bars indicate SEM.

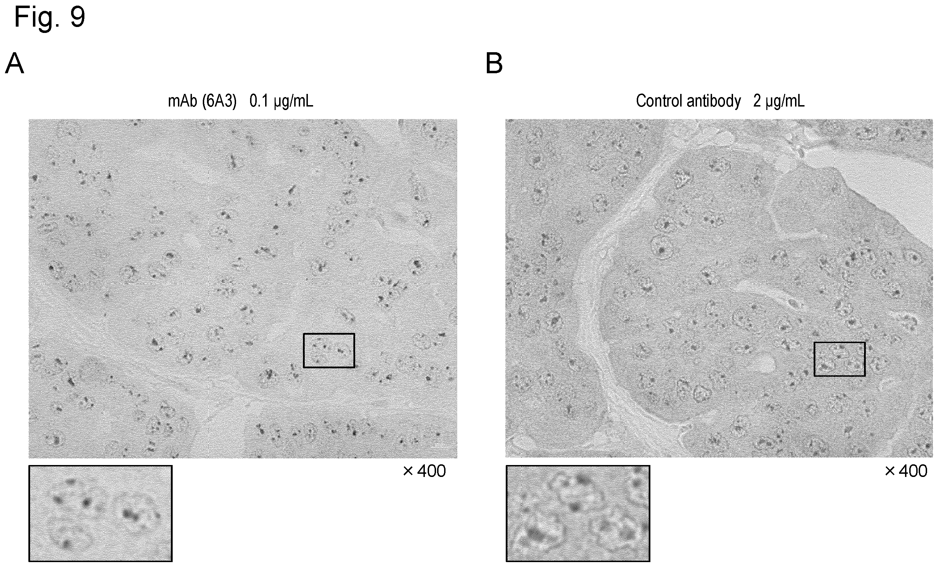

[Figure 9] Figure 9 shows representative images of immunohistochemical staining in a cancer invasion site (lesion site) of invasive breast ductal carcinoma. Figure 9A is an image (at a magnification of ×400) of a cancer invasion site (lesion site) stained with mAb (6A3) at 0.1 µg/mL. The enlarged image of the area shown by the black frame in the image is shown at the bottom left. Figure 9B is an image (at a magnification of ×400) of a cancer invasion site (lesion site) stained with a control antibody at 2 µg/mL. The enlarged image of the area shown by the black frame in the image is shown at the bottome left.

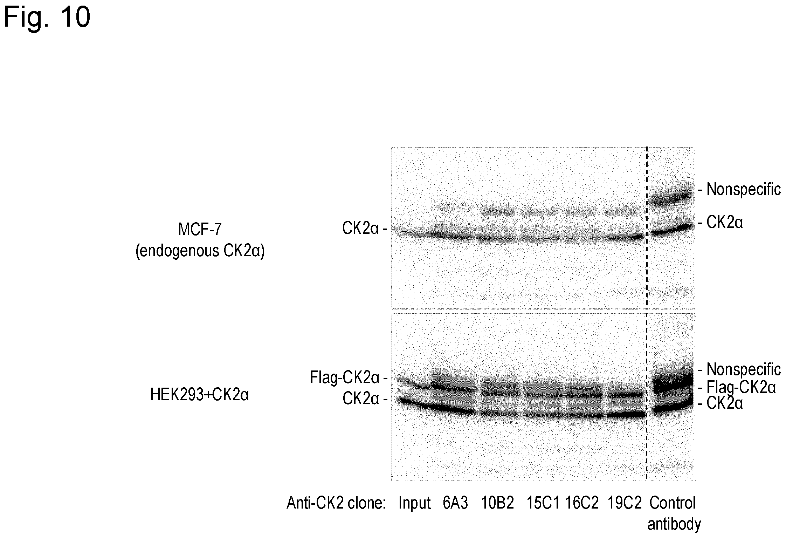

[Figure 10] Figure 10 shows the results of Western blotting for immunoprecipitates of either an MCF-7 cell line, or HEK293 cells expressing Flag-CK2α protein, regarding IgGs purified from a plurality of CK2 antibody clones which were established in addition to 6A3. The upper panel shows the results of detection of the endogenous CK2α protein in the MCF-7 cell line. The bottom panel shows the results of detection of the endogenous CK2α protein and the Flag-CK2α protein in HEK293 cells expressing the Flag-CK2α-protein.

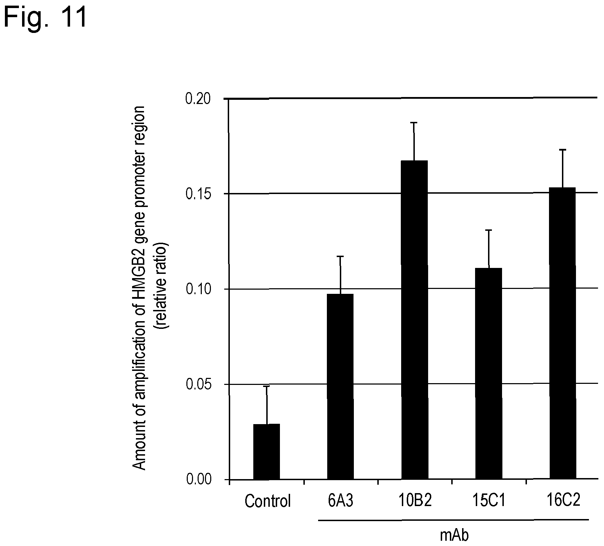

[Figure 11] Figure 11 shows the results of ChIP-qPCR targeting the HMGB2 gene locus for the fractions obtained by chromatin immunoprecipitation with IgGs purified from a plurality of CK2 antibody clones established in addition to 6A3. "Control" shows the lane loaded with a chromatin fraction which was fractionated in the same manner to which IgG was not added

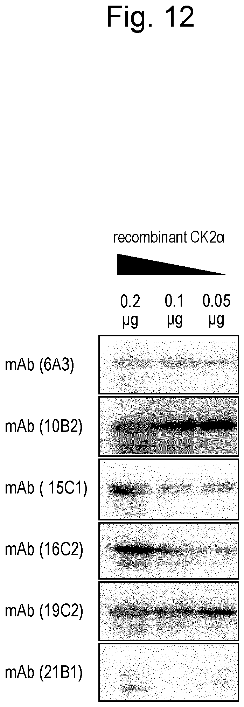

[Figure 12] Figure 12 shows the results of Western blotting for a purified product of the recombinant CK2α protein.

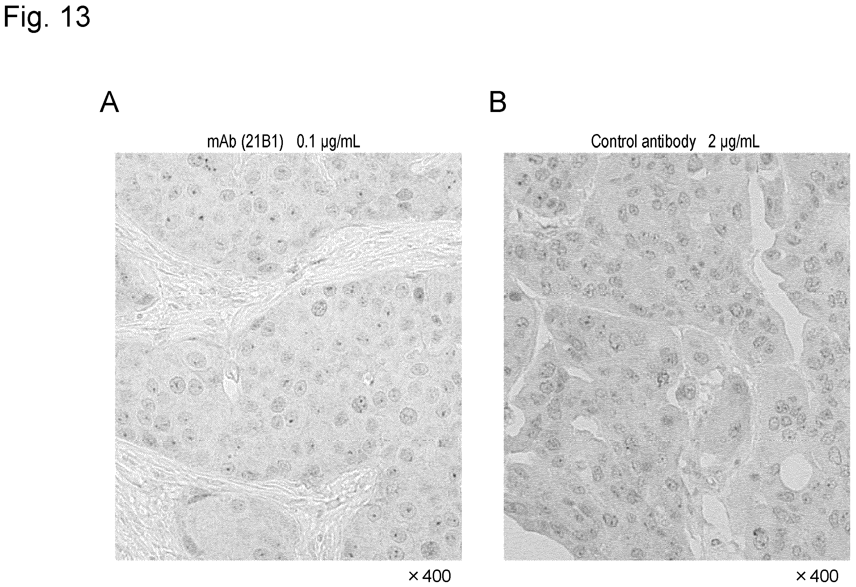

[Figure 13] Figure 13 shows representative images of immunohistochemical staining in a cancer invasion site (lesion site) of invasive breast ductal carcinoma. Figure 13A is an image (at a magnification of ×400) of a cancer invasion area (lesion site) stained with mAb (21B1) at 0.1 µg/mL. Figure 13B is an image (at a magnification of ×400) of a cancer invasion site (lesion site) stained with a control antibody at 2 µg/mL.

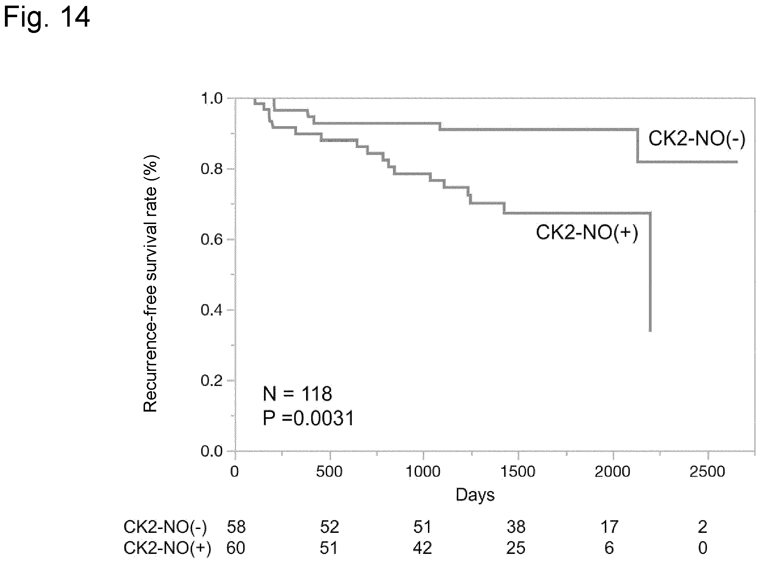

[Figure 14] Figure 14 shows the recurrence-free survival rate of the CK2α nucleolus staining positive group ("CK2-NO(+)" in the figure) and the CK2α nucleolus staining negative group ("CK2-NO(-)" in the figure) based on the results of histochemical staining with the 6A3 clone in an FFPE sample from a primary lung adenocarcinoma patient subjected to surgical resection.

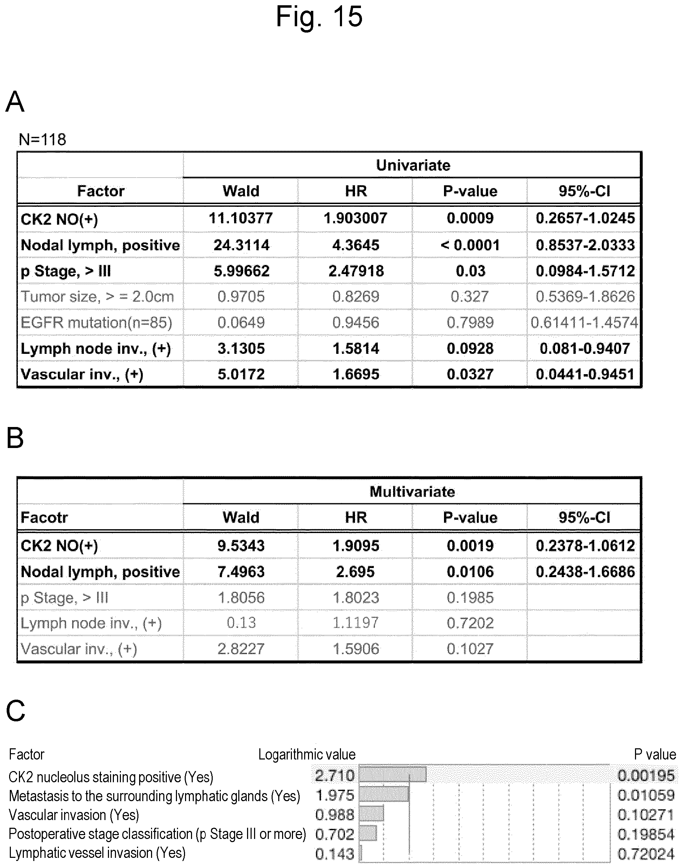

[Figure 15] Figure 15 shows the results of univariate analysis and multivariate analysis based on the results of histochemical staining with the 6A3 clone for an FFPE sample from a primary lung adenocarcinoma patient. Figure 15A shows the results of the univariate analysis. Figure 15B shows the results of the multivariate analysis. Figure 15C shows the results of analyzing the efficacy with respect to determination of recurrence-free survival in the multivariate analysis.

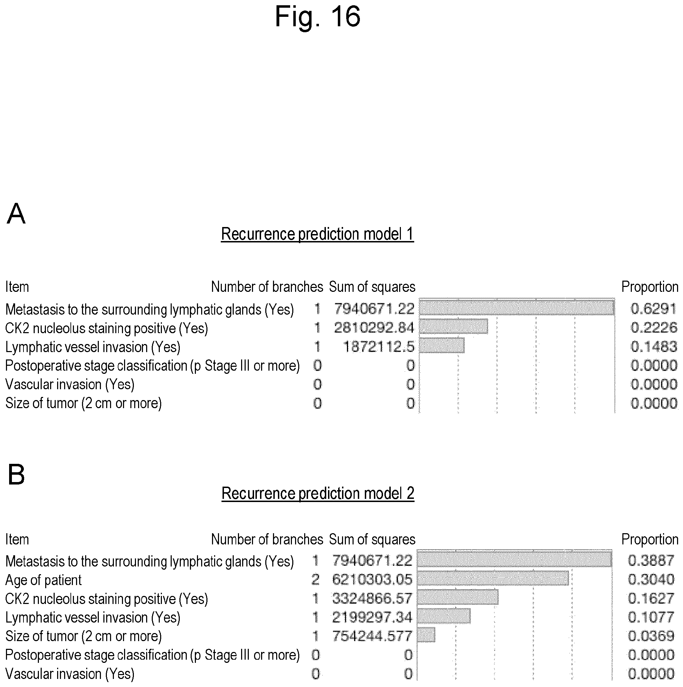

[Figure 16] Figure 16 shows the results of analyzing variables that predict time before/until recurrence using two recurrence prediction models. Figure 16A shows the results of the recurrence prediction model 1 based on six variables. Figure 16B shows the results of the recurrence prediction model 2 based on seven variables additionally including age.

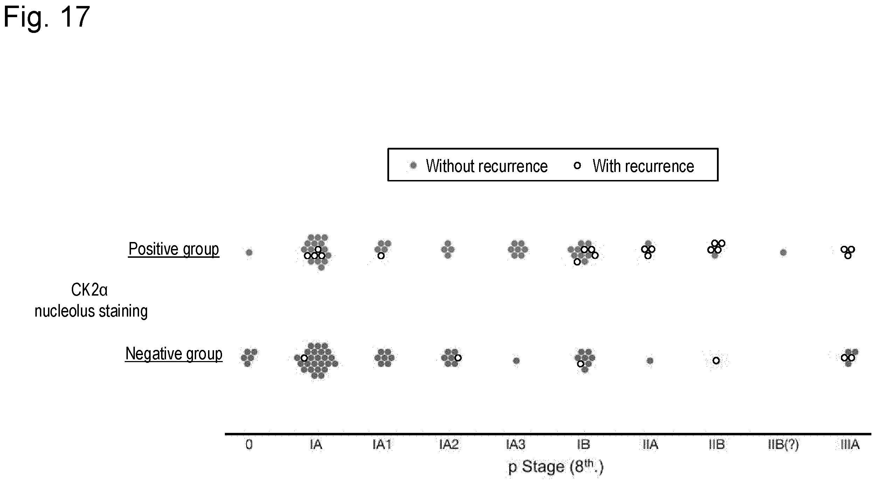

[Figure 17] Figure 17 shows primary lung adenocarcinoma patients at Stages I to III, classified according to the stage and the evaluation of CK2α staining, and distinguished by the presence and absence of recurrence.

### Description of Embodiments

### (Definition of Terms)

As used herein, the "CK2α protein" refers to the α subunit of Casein kinase 2 (CK2). The CK2α protein is also referred to as Casein kinase 2 alpha 1, Casein kinase II subunit alpha, the CK2α1 protein, or the CSNK2A1 protein. The CK2α protein constitutes CK2, which is a tetramer, together with the CK2α' protein and the CK2β protein.

Casein kinase 2 is one type of a serine/threonine kinase and is known to be involved in *e.g.*, pro-survival pathway. Casein kinase 2 is known to function as a tetramer composed of an α subunit (CK2α protein), an α' subunit (CK2α' protein), and two β subunits (CK2β proteins). The α subunit (CK2α protein) and the α' subunit (CK2α' protein) are known to function as the catalytic subunits of Casein kinase 2.

Herein, the CK2α protein or a fragment thereof can be a biomarker for predicting the prognosis of a cancer patient. For example, when the patient is a human, a human CK2α protein or a fragment thereof can be the biomarker.

WO2021/132544 discloses that the CK2α protein in the nucleolus can be a biomarker for predicting the prognosis of a cancer patient such as a breast cancer patient. Specifically, immunohistochemical staining of the CK2α protein was performed on formalin-fixed, paraffin-embedded specimens of breast cancer tissue resected from primary breast cancer patients to whom radical resection was performed. The results revealed that CK2α protein is present throughout the cell in normal cells, but many cases of high expression in the nucleus are observed in breast cancer cells, that CK2α protein is localized to the nucleolus in the nucleus, in some breast cancer patients (nearly and fewer than 40%), and that when the intranuclear expression level and nucleolus staining level of CK2α protein are higher, the percentage of the cases at higher stages of breast cancer is higher. Furthermore, the results of evaluating the prognosis of primary breast cancer patients revealed that the nucleolar localization of the CK2α protein represents a high relative risk regarding both recurrence and prognosis of life, and that the presence or absence of the nucleolar localization of the CK2α protein is a strong factor for predicting recurrence. In addition, tissues of various cancers other than breast cancer were also examined for the localization of the CK2α protein. As a result, it was found that the CK2α protein can localize in the nucleolus in cancers in general, including breast cancer, uterine cancer, esophageal cancer, gastric cancer, biliary tract cancer, pancreatic cancer, liver cancer, renal cancer, colorectal cancer (rectal cancer and colon cancer), bladder cancer, lung cancer (lung adenocarcinoma and squamous cell lung cancer), thyroid cancer, and glioma.

Unless otherwise specified, a "marker" as used herein means a biomarker consisting of the CK2α protein or a fragment thereof, or a biomarker consisting of the CK2α protein or a fragment thereof in the nucleolus.

As used herein, the type of "cancer" is not limited, and examples thereof include adenocarcinoma, squamous cell carcinoma, small cell carcinoma, and large cell carcinoma. Specific examples of cancer types include malignant melanoma, oral cavity cancer, laryngeal cancer, pharyngeal cancer, thyroid cancer, lung cancer, breast cancer, esophageal cancer, gastric cancer, colorectal cancer (including colon cancer and rectal cancer), small bowel cancer, bladder cancer, prostate cancer, testicular cancer, uterine cancer, cervical cancer, endometrial cancer, ovarian cancer, gastric cancer, renal cancer, liver cancer, pancreatic cancer, biliary tract cancer (including gallbladder cancer and bile duct cancer), brain tumor, head and neck cancer, mesothelioma, osteosarcoma, glioma, a childhood tumor including neuroblastoma, leukemia, and lymphoma. The cancer is preferably breast cancer, uterine cancer, esophageal cancer, gastric cancer, pancreatic cancer, liver cancer, biliary tract cancer (for example, gallbladder or bile duct cancer), renal cancer, colorectal cancer (for example, rectal cancer and colon cancer), bladder cancer, lung cancer (for example, lung adenocarcinoma or squamous cell lung cancer), thyroid cancer, or glioma (for example, astrocytoma), and more preferably breast cancer.

As used herein, the type of "breast cancer" is not limited, and examples thereof include non-invasive breast ductal carcinoma, invasive breast ductal carcinoma, invasive lobular carcinoma, non-invasive lobular carcinoma, and special types of carcinoma such as medullary carcinoma, mucinous carcinoma, or tubular carcinoma.

As used herein, "prognosis" refers to a predicted course (for example, the presence or absence of recurrence, or survival or death) in a cancer patient. "Prediction of prognosis" may be a prediction of recurrence risk (for example, recurrence-free survival rate), length of survival, or survival rate at a certain time after surgery (for example, at the time after 1, 2, 3, 4, 5, 10, 15, or 20 years or longer), recurrence-free survival rate (RFS), or disease-free survival rate (DFS). In one embodiment, prediction of prognosis includes prediction of recurrence risk (for example, recurrence-free survival rate). As used herein, recurrence-free survival rate is the proportion of patients free of recurrence of cancer, such as cancer associated with a first cancer, and disease-specific survival rate is the proportion of patients free of death associated with a first cancer. Prediction of prognosis can also be determination, evaluation, or diagnosis of prognosis, or an assistance thereof.

Specific examples of a CK2α protein include a human-derived CK2α (human CK2α) protein comprising or consisting of the amino acid sequence of SEQ ID NO: 2.

The CK2α protein also encompasses a CK2α variant having a functionally comparable activity to the CK2α protein represented by SEQ ID NO: 2, as well as a CK2α orthologue of other species. Specific examples thereof include an amino acid sequence in which one or several amino acids are deleted, substituted, or added in the amino acid sequence of SEQ ID NO: 2, or a CK2α protein having 80% or more, 90% or more, 95% or more, 97% or more, 98% or more, or 99% or more amino acid identity to the amino acid sequence of SEQ ID NO: 2.

As used herein, "several" means, for example, 2 to 10, 2 to 7, 2 to 5, 2 to 4, or 2 to 3. As for an amino acid substitution, a conservative amino acid substitution is preferable. A "conservative amino acid substitution" refers to a substitution between amino acids having similar properties such as charge, side chain, polarity, and aromaticity. Amino acids with similar properties can be classified into, for example, a basic amino acid (arginine, lysine, histidine), an acidic amino acid (aspartic acid, glutamic acid), an uncharged polar amino acid (glycine, asparagine, glutamine, serine, threonine, cysteine, tyrosine), a non-polar amino acid (leucine, isoleucine, alanine, valine, proline, phenylalanine, tryptophan, methionine), a branched-chain amino acid (leucine, valine, isoleucine), and an aromatic amino acid (phenylalanine, tyrosine, tryptophan, histidine).

As used herein, "amino acid identity" refers to the proportion (%) of identical amino acid residues between two amino acid sequences relative to the total amino acid residues of a CK2α protein comprising the amino acid sequence of SEQ ID NO: 2 when two amino acid sequences are aligned and gaps are introduced if necessary to achieve the highest degree of amino acid identity between the two amino acid sequences. Amino acid identity can be calculated using a protein search system by BLAST or FASTA. For details of methods for determining identity, see, for example, Altschul et al, Nuc. Acids. Res. 25, 3389-3402, 1977 and Altschul et al, J. Mol. Biol. 215, 403-410, 1990.

A CK2α protein is encoded by a CK2α gene. Specific examples of the CK2α gene include a human CK2α gene encoding the human CK2α protein comprising the amino acid sequence of SEQ ID NO: 2. More specific examples of the CK2α include a gene comprising or consisting of the base sequence of SEQ ID NO: 1.

The CK2α gene encompasses a CK2α gene encoding a CK2α variant having functionally comparable activity to a CK2α protein encoded by the CK2α gene of SEQ ID NO: 1, or a CK2α gene encoding a CK2α ortholog of other species. Specifically, the CK2α gene encompasses a CK2α gene having the base sequence of SEQ ID NO: 1 in which one or several bases are deleted, substituted, or added, or having 80% or more, 90% or more, 95% or more, 97% or more, 98% or more, or 99% or more base identity to the base sequence of SEQ ID NO: 1. Furthermore, the CK2α gene encompasses a gene that comprises a base sequence that hybridizes under high-stringent conditions with a nucleic acid fragment comprising a portion of a complementary base sequence to the base sequence of SEQ ID NO: 1 and that encodes a protein having functionally comparable activity to a CK2α protein.

As used herein, "base identity" refers to the proportion (%) of identical bases between two base sequences relative to the total bases of the CK2α gene comprising the sequence of SEQ ID NO: 2, when the two base sequences are aligned and gaps are introduced if necessary to achieve the highest degree of base identity between the two.

As used herein, "hybridize under high-stringent conditions" refers to hybridization and washing under low salt concentration and/or high temperature conditions. For example, an incubation is performed with a probe in 6×SSC, 5×Denhardt's reagent, 0.5% SDS, 100 µg/mL denatured fragmented salmon sperm DNA at from 65°C to 68°C, followed by washing in 2×SSC, 0.1% SDS washing solution starting at room temperature, lowering the salt concentration in the washing solution to 0.1×SSC, and raising the temperature to 68°C until no background signal is detected. The conditions for high-stringent hybridization can be found in Green, M.R. and Sambrook, J., 2012, Molecular Cloning: A Laboratory Manual Fourth Ed., Cold Spring Harbor Laboratory Press, Cold Spring Harbor, New York for reference.

The base sequence information of such a CK2α gene can be searched from public databases (GenBank, EMBL, DDBJ). For example, based on the known base sequence information of the CK2α gene of SEQ ID NO: 1, genes having a high base identity can be searched and obtained from the databases.

As used herein, a "fragment" of a CK2α protein is a peptide fragment comprising or consisting of a portion of an amino acid sequence constituting the CK2α protein, which can be identified as a fragment of the CK2α protein from the amino acid sequence constituting the fragment. For example, a "fragment" may be 5 or more, 8 or more, 10 or more, 20 or more, 30 or more, 40 or more, or 50 or more consecutive amino acid residues of the full-length amino acid sequence of a CK2α protein, or a peptide consisting of 200 or less, 150 or less, 120 or less, 100 or less, or 80 or less consecutive amino acid residues. For example, a "fragment" may be a peptide consisting of from 5 to 200, from 10 to 120, or from 50 to 80 consecutive amino acid residues.

As used herein, a "nucleolus" refers to a region of high molecular density in the nucleus of a eukaryotic cell where rRNA transcription and ribosome production take place. A nucleolus is generally observable under a light microscope. Usually, one nucleolus is observed within a nucleus, but a plurality of nucleoli may be observed.

### (Anti-CK2α Antibody or Fragment Thereof)

In one aspect, the present invention relates to an anti-CK2α antibody or a fragment thereof. An anti-CK2α antibody or a fragment thereof of the present invention can predict the prognosis of a cancer patient by detecting a CK2α protein or a peptide fragment thereof that can be localized to the nucleolus in a highly malignant cancer.

### (1) Anti-CK2α Antibody

As used herein, an "anti-CK2α antibody" refers to an antibody that exhibits immunoresponsiveness to a CK2α protein or a peptide fragment thereof.

The species of the origin of an anti-CK2α antibody of the present invention is not particularly limited. The antibody is preferably derived from a bird or a mammal. Examples thereof include *e.g*., a chicken, an ostrich, a mouse, a rat, a guinea pig, a rabbit, a goat, a donkey, a sheep, a camel, a horse, or a human.

An anti-CK2α antibody of the present invention is a monoclonal antibody. As used herein, a "monoclonal antibody" refers to a single species of immunoglobulin which comprises a framework region (hereinafter referred to as an "FR") and a complementarity determining region (hereinafter referred to as a "CDR"), and which can specifically bind to an antigen and recognize the antigen, or a recombinant antibody or a synthetic antibody encompassing at least one set of a light chain variable region (V_{L} region) and a heavy chain variable region (V_{H} region) that are contained in an immunoglobulin.

In a case where the anti-CK2α antibody is composed of an immunoglobulin molecule, the immunoglobulin can be of any class (for example, IgG, IgE, IgM, IgA, IgD, or IgY) or any subclass (for example, IgG1, IgG2, IgG3, IgG4, IgA1, or IgA2).

An epitope to be recognized in a CK2α protein or a peptide fragment thereof by the anti-CK2α antibody of the present invention is an epitope comprised specifically in the CK2α protein. This epitope is preferably not comprised in a CK2α' protein.

Specific examples of an anti-CK2α antibody that recognizes the above-described epitope include a mouse anti-CK2α monoclonal antibody clone 6A3 (herein referred to as "mAb (6A3)") in the Examples described below. In this mAb (6A3), the heavy chain variable region consists of the amino acid sequence of SEQ ID NO: 11, and the light chain variable region consists of the amino acid sequence of SEQ ID NO: 12. According to the rule of Kabat (Kabat E.A., et al., 1991, Sequences of proteins of immunological interest, Vol. 1, eds. 5, NIH publication), in the heavy chain variable region of mAb (6A3), CDR1 (HCDR1) consists of the amino acid sequence of SEQ ID NO: 5, CDR2 (HCDR2) consists of the amino acid sequence of SEQ ID NO: 6, and CDR3 (HCDR3) consists of the amino acid sequence (FV) of SEQ ID NO: 7. Additionally, in the light chain variable region of an mAb (6A3), CDR1 (LCDR1) consists of the amino acid sequence of SEQ ID NO: 8, CDR2 (LCDR2) consists of the amino acid sequence of SEQ ID NO: 9, and CDR3 (LCDR3) consists of the amino acid sequence of SEQ ID NO: 10. The amino acid sequences of SEQ ID NOs: 5 to 12 are shown in the following Table 1.

### [Table 1]

**Table 1: Amino acid sequence of CK2α monoclonal antibody**

| Region | Amino acid sequence | SEQ ID NO |
|---|---|---|
| Heavy chain variable region | MEWSWVFLFLLSVIAGVQSQVQLQQSGAELVRPGASV TLSCKASGYKFTDYQMHWVKQTPVHGLEWIGVIDPGT GGTAYNQKFKGKAILTADKSSSTAYMELRSLTSEDSA VYYCTGFVWGTGTTVTVSSAKTTPPSVYPLAPGCGDT TGSSVTLGCLVKGYFPESVT | 11 |
| Light chain variable region | MDMRAPAQIFGFLLLLFPGSRCDIQMTQSPSSLSASL GERVSLTCRASQDIGSSLNWLQQEPDGTIKRLIYATS SLDSGVPKRFSGSRSGSDYSLTISSLESEDFVDYYCL QYAIFPYTFGGGTKLEIKRADAAPTVSIFPPSSEQLT SGGASVVCFLN | 12 |
| Heavy chain CDR1 | DYQMH | 5 |
| Heavy chain CDR2 | VIDPGTGGTAYNQKFKG | 6 |
| Heavy chain CDR3 | FV | 7 |
| Light chain CDR1 | RASQDIGSSLN | 8 |
| Light chain CDR2 | ATSSLDS | 9 |
| Light chain CDR3 | LQYAIFPYT | 10 |

Other specific examples of an anti-CK2α antibody that recognizes the above-described epitope include mouse anti-CK2α monoclonal antibody clones 10B2 (herein referred to as "mAb (10B2)"), 15C1 (herein referred to as "mAb (15C1)"), 16C2 (herein referred to as "mAb (16C2)"), 19C2 (herein referred to as "mAb (19C2)"), and 21B1 (herein referred to as "mAb (21B 1)") in the Examples described below.

In mAb (10B2), the heavy chain variable region consists of the amino acid sequence of SEQ ID NO: 15, and the light chain variable region consists of the amino acid sequence of SEQ ID NO: 16. According to the rule of Kabat, in the heavy chain variable region of mAb (10B2), CDR1 (HCDR1) consists of the amino acid sequence of SEQ ID NO: 17, CDR2 (HCDR2) consists of the amino acid sequence of SEQ ID NO: 18, and CDR3 (HCDR3) consists of the amino acid sequence of SEQ ID NO: 19. Additionally, in the light chain variable region of mAb (10B2), CDR1 (LCDR1) consists of the amino acid sequence of SEQ ID NO: 20, CDR2 (LCDR2) consists of the amino acid sequence of SEQ ID NO: 21, and CDR3 (LCDR3) consists of the amino acid sequence of SEQ ID NO: 22.

In mAb (15C1), the heavy chain variable region consists of the amino acid sequence of SEQ ID NO: 23, and the light chain variable region consists of the amino acid sequence of SEQ ID NO: 24. According to the rule of Kabat, in the heavy chain variable region of mAb (15C1), CDR1 (HCDR1) consists of the amino acid sequence of SEQ ID NO: 25, CDR2 (HCDR2) consists of the amino acid sequence of SEQ ID NO: 26, and CDR3 (HCDR3) consists of the amino acid sequence of SEQ ID NO: 27. Additionally, in the light chain variable region of mAb (15C1), CDR1 (LCDR1) consists of the amino acid sequence of SEQ ID NO: 28, CDR2 (LCDR2) consists of the amino acid sequence of SEQ ID NO: 29, and CDR3 (LCDR3) consists of the amino acid sequence of SEQ ID NO: 30.

In mAb (16C2), the heavy chain variable region consists of the amino acid sequence of SEQ ID NO: 31, and the light chain variable region consists of the amino acid sequence of SEQ ID NO: 32. According to the rule of Kabat, in the heavy chain variable region of mAb (16C2), CDR1 (HCDR1) consists of the amino acid sequence of SEQ ID NO: 33, CDR2 (HCDR2) consists of the amino acid sequence of SEQ ID NO: 34, and CDR3 (HCDR3) consists of the amino acid sequence of SEQ ID NO: 35. Additionally, in the light chain variable region of mAb (16C2), CDR1 (LCDR1) consists of the amino acid sequence of SEQ ID NO: 36, CDR2 (LCDR2) consists of the amino acid sequence of SEQ ID NO: 37, and CDR3 (LCDR3) consists of the amino acid sequence of SEQ ID NO: 38.

In mAb (19C2), the heavy chain variable region consists of the amino acid sequence of SEQ ID NO: 39, and the light chain variable region consists of the amino acid sequence of SEQ ID NO: 40. According to the rule of Kabat, in the heavy chain variable region of mAb (19C2), CDR1 (HCDR1) consists of the amino acid sequence of SEQ ID NO: 41, CDR2 (HCDR2) consists of the amino acid sequence of SEQ ID NO: 42, and CDR3 (HCDR3) consists of the amino acid sequence of SEQ ID NO: 43. Additionally, in the light chain variable region of mAb (19C2), CDR1 (LCDR1) consists of the amino acid sequence of SEQ ID NO: 44, CDR2 (LCDR2) consists of the amino acid sequence of SEQ ID NO: 45, and CDR3 (LCDR3) consists of the amino acid sequence of SEQ ID NO: 46.

In mAb (21B 1), the heavy chain variable region consists of the amino acid sequence of SEQ ID NO: 47, and the light chain variable region consists of the amino acid sequence of SEQ ID NO: 48. According to the rule of Kabat that, in the heavy chain variable region of mAb (21B1), CDR1 (HCDR1) consists of the amino acid sequence of SEQ ID NO: 49, CDR2 (HCDR2) consists of the amino acid sequence of SEQ ID NO: 50, and CDR3 (HCDR3) consists of the amino acid sequence of SEQ ID NO: 51. Additionally, in the light chain variable region of mAb (21B 1), CDR1 (LCDR1) consists of the amino acid sequence of SEQ ID NO: 52, CDR2 (LCDR2) consists of the amino acid sequence of SEQ ID NO: 53, and CDR3 (LCDR3) consists of the amino acid sequence of SEQ ID NO: 54.

Examples of a nucleic acid (nucleotide) encoding the amino acid sequence of SEQ ID NO: 11 corresponding to the heavy chain variable region of mAb (6A3) include a nucleic acid consisting of the base sequence of SEQ ID NO: 13. In addition, examples of a nucleic acid encoding the amino acid sequence of SEQ ID NO: 12 corresponding to the light chain variable region of mAb (6A3) include a nucleic acid consisting of the base sequence of SEQ ID NO: 14.

Examples of a nucleic acid (nucleotide) encoding the amino acid sequence of SEQ ID NO: 15 corresponding to the heavy chain variable region of mAb (10B2) include a nucleic acid consisting of the base sequence of SEQ ID NO: 55. In addition, examples of a nucleic acid encoding the amino acid sequence of SEQ ID NO: 16 corresponding to the light chain variable region of mAb (10B2) include a nucleic acid consisting of the base sequence of SEQ ID NO: 56.

Examples of a nucleic acid (nucleotide) encoding the amino acid sequence of SEQ ID NO: 23 corresponding to the heavy chain variable region of mAb (15C1) include a nucleic acid consisting of the base sequence of SEQ ID NO: 57. In addition, examples of a nucleic acid encoding the amino acid sequence of SEQ ID NO: 24 corresponding to the light chain variable region of mAb (15C1) include a nucleic acid consisting of the base sequence of SEQ ID NO: 58.

Examples of a nucleic acid (nucleotide) encoding the amino acid sequence of SEQ ID NO: 31 corresponding to the heavy chain variable region of mAb (16C2) include a nucleic acid consisting of the base sequence of SEQ ID NO: 59. In addition, examples of a nucleic acid encoding the amino acid sequence of SEQ ID NO: 32 corresponding to the light chain variable region of mAb (16C2) include a nucleic acid consisting of the base sequence of SEQ ID NO: 60.

Examples of a nucleic acid (nucleotide) encoding the amino acid sequence of SEQ ID NO: 39 corresponding to the heavy chain variable region of mAb (19C2) include a nucleic acid consisting of the base sequence of SEQ ID NO: 61. In addition, examples of a nucleic acid encoding the amino acid sequence of SEQ ID NO: 40 corresponding to the light chain variable region of mAb (19C2) include a nucleic acid consisting of the base sequence of SEQ ID NO: 62.

Examples of a nucleic acid (nucleotide) encoding the amino acid sequence of SEQ ID NO: 47 corresponding to the heavy chain variable region of mAb (21B 1) include a nucleic acid consisting of the base sequence of SEQ ID NO: 63. In addition, examples of a nucleic acid encoding the amino acid sequence of SEQ ID NO: 48 corresponding to the light chain variable region of mAb (21B1) include a nucleic acid consisting of the base sequence of SEQ ID NO: 64.

A "recombinant antibody" refers to a chimeric antibody or a humanized antibody. A "chimeric antibody" is an antibody produced by combining the amino acid sequences of antibodies derived from different animals, in which the constant region (C region) of one of the antibodies is substituted with the C region of another antibody. Examples thereof include an antibody in which the C region of a mouse monoclonal antibody is substituted with the C region of a human antibody. Specific examples thereof include an antibody obtained by substituting the heavy chain variable region of a human antibody to any antigen with the heavy chain variable region consisting of the amino acid sequence of SEQ ID NO: 11 in the above-described mAb (6A3), and substituting the light chain variable region of the human antibody with the light chain variable region consisting of the amino acid sequence of SEQ ID NO: 12. As a result, immunoreaction to the same antibody in the human body can be reduced. A "humanized antibody" is a mosaic antibody obtained by substituting the CDRs in a human antibody with the CDRs in an antibody derived from a mammal other than a human. The variable region (V region) of an immunoglobulin molecule is composed of four FRs (FR1, FR2, FR3, and FR4) and three CDRs (CDR1, CDR2, and CDR3), which are linked in the order of FR1-CDR1-FR2-CDR2-FR3-CDR3-FR4 from the N-terminal side. The FR among them is a relatively conserved region constituting the backbone of a variable region, and the CDRs contribute directly to the antigen binding specificity of an antibody. For example, a humanized antibody can be constructed as a human antibody inheriting the antigen binding specificity of mAb (6A3) that is a mouse antibody, by substituting one set of CDR1, CDR2, and CDR3 in the light chain or heavy chain of the mouse-derived mAb (6A3) with one set of CDR1, CDR2, and CDR3, respectively, of the light chain or heavy chain of a human antibody for any antigen. Specific examples thereof include an antibody obtained by substituting CDR1 consisting of the amino acid sequence of SEQ ID NO: 5, CDR2 consisting of the amino acid sequence of SEQ ID NO: 6, and CDR3 consisting of the amino acid sequence of SEQ ID NO: 7, which are derived from the heavy chain in the above-described mAb (6A3), with CDR1, CDR2, and CDR3, respectively, of the heavy chain of a human antibody, and additionally, CDR1 consisting of the amino acid sequence of SEQ ID NO: 8, CDR2 consisting of the amino acid sequence of SEQ ID NO: 9, and CDR3 consisting of the amino acid sequence of SEQ ID NO: 10, which are derived from the light chain in the above-described mAb (6A3) with CDR1, CDR2, and CDR3, respectively, of the light chain of a human antibody. Such a humanized antibody is derived from a human antibody except CDRs, and hence, can reduce immunoreaction to the same antibody in the human body, better than a chimeric antibody.

A "synthetic antibody" refers to an antibody synthesized chemically or using a recombinant DNA method. Examples thereof include, *e.g*., an antibody newly synthesized using a recombinant DNA method. Specific examples include an scFv (single chain Fragment of variable region: single chain antibody), diabody, triabody, and tetrabody. In an immunoglobulin molecule, one set of variable regions (a light chain variable region V_{L} and a heavy chain variable region V_{H}) that form a functional antigen binding site are located on the separate polypeptide chains, namely, a light chain and a heavy chain. In an immunoglobulin molecule, an scFv is a synthetic antibody that has a molecular weight of approximately 35 kDa or less, and has a structure in which a V_{L} and a V_{H} are linked via a flexible linker having a sufficient length, and are encompassed in one polypeptide chain. In an scFv, one set of variable regions can self-assemble with each other to form one functional antigen binding site. An scFv can be obtained by incorporating a recombinant DNA encoding the scFV into a vector using a known technology, and expressing it. A diabody is a molecule having a structure based on the dimeric structure of an scFv (Holliger et al., 1993, Proc. Natl. Acad. Sci. USA 90: 6444-6448). For example, when the length of the above-described linker is shorter than approximately 12 amino acid residues, two variable regions in an scFv cannot self-assemble, but allowing two scFvs to interact to form a diabody enables the V_{L} of one scFv to assemble with the V_{H} of the other scFv to form two functional antigen binding sites. Furthermore, adding a cysteine residue to the C-terminus of an scFv allows a disulfide bond between two scFvs, making it possible to form a stable diabody. In this way, a diabody is a divalent antibody fragment. A triabody and a tetrabody are a trivalent and a tetravalent antibody respectively, which have a trimeric and a tetrameric structure respectively based on the scFv structure in the same manner as a diabody. The diabody, triabody, and tetrabody may be a multispecific antibody. A "multispecific antibody" refers to a multivalent antibody, that is, an antibody having a plurality of antigen binding sites in one molecule, in which the individual antigen binding sites bind to different epitopes. Examples thereof include a bispecific antibody that is a diabody in which the individual antigen binding sites bind to different epitopes. Specific examples thereof, in the case of an anti-CK2α antibody of the present invention, include a diabody in which one of the antigen binding sites comprises a heavy chain variable region consisting of the amino acid sequence of SEQ ID NO: 11 and a light chain variable region consisting of the amino acid sequence of SEQ ID NO: 12, and in which the other antigen binding site binds to a different epitope.

An anti-CK2α antibody of the present invention can be modified. As used herein, the term "modified" comprises functional modification necessary for antigen-specific binding avidity, such as glycosylation, and labeling modification necessary for antibody detection.

Glycosylation modification on an anti-CK2α antibody is performed for adjusting the affinity of the anti-CK2α antibody to a CK2α protein or a peptide fragment thereof as a target. Specific examples thereof include a modification in the FR of an anti-CK2α antibody in which a substitution is introduced into the amino acid residue constituting the glycosylation for removing the glycosylation site, thereby causing the loss of glycosylation at that site.

Examples of the labeling of an anti-CK2α antibody include labeling with a fluorescent dye (FITC, rhodamine, Texas red, Cy3, or Cy5), fluorescent protein (for example, PE, APC, or GFP), enzyme (for example, horseradish peroxidase, alkaline phosphatase, or glucose oxidase), radioisotope (for example, ³H, ¹⁴C, or ³⁵S), or biotin or (strept)avidin.

An anti-CK2α antibody of the present invention preferably has a dissociation constant of 10⁻⁷ M or less with a CK2α protein, and for example, preferably has a high affinity of 10⁻⁸ M or less, more preferably 10⁻⁹ M or less, particularly preferably 10⁻¹⁰ M or less. The above-described dissociation constant can be measured using a technology known in the art. For example, a measurement may be carried out using a Biacore system (GE Healthcare BioSciences Corp.) using rate evaluation kit software.

### (2) Fragment Thereof

As used herein, a "fragment thereof' refers to an antibody fragment that consists of part of an anti-CK2α antibody, and exhibits immunoresponsiveness to a CK2α protein or a fragment thereof in the same manner as the anti-CK2α antibody. Examples thereof include, *e.g*., a Fab; F(ab')₂; Fab'; Fv fragment; Fv fragment stabilized by a disulfide bond (dsFv); (dsFv)₂; bispecific dsFv (dsFv-dsFv'); diabody stabilized by a disulfide bond (ds diabody); single chain antibody molecule (scFv); dimeric scFv (divalent diabody); multispecific antibody; heavy chain antibody such as a camelized single domain antibody (camelized antibody; VHH antibody); nanobody; domain antibody; and divalent domain antibody.

A Fab is an antibody fragment produced by cleavage of an IgG molecule by papain at the N-terminus side of the disulfide bond in the hinge region, and is composed of C_{H}1 flanking with V_{H} among three domains (C_{H}1, C_{H}2, and C_{H}3) constituting the H chain constant region (heavy chain constant region: hereinafter referred to as C_{H}), V_{H}, and a full-length L chain.

A F(ab')₂ is a dimer of a Fab' produced by cleavage of an IgG molecule by pepsin at the C-terminus side of the disulfide bond in the hinge region. A Fab' has a slightly longer H chain than a Fab by the length of the hinge region contained in the Fab', but has a structure substantially comparable to the structure of a Fab. A Fab' can be obtained by reducing a F(ab')₂ under mild conditions, and cleaving the disulfide linkage in the hinge region. These antibody fragments all encompass an antigen binding site, and hence, have an ability to specifically bind to an antigen epitope.

### (3) Production of Anti-CK2α Antibody

An anti-CK2α antibody of the present invention can be obtained by a common method in the art. In addition, if the amino acid sequence of a monoclonal antibody is known, the antibody can be prepared on the basis of the amino acid sequence using a chemical synthesis method or a recombinant DNA technology. Furthermore, a monoclonal antibody can be obtained from a hybridoma that produces the antibody.

An antigen polypeptide or an antigen peptide that can be used as an immunogen for an anti-CK2α antibody of the present invention is any part or the full length of a CK2α protein (hereinafter referred to as a "CK2α antigen polypeptide"). For example, one example of an antigen polypeptide that can be used as an immunogen for an anti-CK2α antibody of the present invention is a full-length human CK2α protein consisting of the amino acid sequence of SEQ ID NO: 2. A CK2α antigen peptide can be prepared, for example, using a chemical synthesis method or a DNA recombinant technology.

In one embodiment, the present invention provides an anti-CK2α antibody or a fragment thereof for predicting the prognosis of a cancer patient.

An anti-CK2α antibody or a fragment thereof of the present invention allows the specific detection of a CK2α protein. High-sensitivity and specific detection can be carried out by using an anti-CK2α antibody or a fragment thereof of the present invention, for example, in immunohistochemical staining, enzyme immunoassay measurement (comprising an ELISA method and an EIA method), Western blotting, radioimmunoassay (RIA), immunoprecipitation, chromatin immunoprecipitation (CHIP), or flow cytometry.

### (Kit for Predicting Prognosis of Cancer Patient)

In one aspect, the present invention relates to a kit for predicting the prognosis of a cancer patient. A kit for predicting the prognosis of a cancer patient according to the present invention comprises, as an essential constituent, the above-described anti-CK2α antibody or an immunoresponsive fragment thereof, and can detect a biomarker consisting of a CK2α protein for predicting the prognosis of a cancer patient. A kit for predicting the prognosis of a cancer patient according to the present invention comprises, as an optional constituent, an antibody (hereinafter referred to as "another antibody for predicting the prognosis") or an immunoresponsive fragment thereof for a biomarker other than a CK2α protein or a peptide fragment thereof for predicting the prognosis of a cancer patient (hereinafter referred to as "another biomarker for predicting the prognosis").

### (1) Essential Constituent

A kit for predicting the prognosis of a cancer patient according to the present invention comprises the above-described anti-CK2α antibody or a fragment thereof as an essential constituent. An anti-CK2α antibody comprised in a kit for predicting the prognosis of a cancer patient according to the present invention may of a single species, or may be of a plurality of species.

### (2) Optional Constituent

A kit for predicting the prognosis of a cancer patient according to the present invention may further comprise, as an optional constituent, one species of or a plurality of species of another antibody or an immunoresponsive fragment thereof for predicting the prognosis. In this connection, another antibody for predicting the prognosis can be any antibody if the antibody can improve the precision of the prediction of the prognosis of a cancer patient when it is used in combination with the above-described anti-CK2α antibody. As such another antibody, an antibody to any biomarker for predicting the prognosis of a cancer can be used. Such a biomarker can be selected from known cancer markers.

A kit for predicting the prognosis of a cancer patient according to the present invention may comprise another reagent necessary for predicting the prognosis of a cancer patient, for example, a known reagent for immunohistochemical staining, ELISA, and Western blotting, examples of which include a labeling reagent, buffer, chromogenic substrate, secondary antibody, and blocking agent, an instrument and a control buffer necessary for testing, and an instruction manual to be used for detection and determination of the results, in addition to the above-described essential constituent.

### (Method for Predicting Prognosis of Cancer Patient)

In one aspect, the present invention relates to a method for predicting the prognosis of a cancer patient.

In one embodiment, cancer is selected from the group consisting of breast cancer, uterine cancer, esophageal cancer, gastric cancer, pancreatic cancer, liver cancer, biliary tract cancer, renal cancer, colorectal cancer, bladder cancer, lung cancer, thyroid cancer, and glioma.

In one embodiment, the present invention combines the marker consisting of a CK2α protein or a fragment thereof with factors such as classification by stage, tumor diameter, presence or absence of lymph node metastasis, and histological grade for predicting the prognosis of a cancer patient.

In one embodiment, cancer is breast cancer, and the marker is combined with at least one, such as two, preferably all three, of the following: classification by stage, classification by hormone receptor expression status, and classification by the HER2 gene and/or protein expression status, for predicting the prognosis of a breast cancer patient. In one embodiment, the above-described marker is combined with other factors such as tumor diameter, presence or absence of lymph node metastasis, and histological grade in addition to or separately from the above-described classification, for predicting the prognosis of a breast cancer patient. Combination with another classification or factor can produce an effect of enabling better prediction of the prognosis.

As used herein, classification by stage is a stage classification based on TNM classification (UICC International Code, L.H. Sobin, M.K. Gospodarowicz and Ch. Wittekind, TNM Classification of Malignant Tumours, 7th edition) of the Union for International Cancer Control (UICC). The above-described TNM classification of the Union for International Cancer Control (UICC) is herein referred to as the UICC-TNM classification. In the UICC-TNM classification, breast cancer is classified into Stages 0, I, II, III, and IV from the lower degree of progression. The UICC-TNM classification classifies the progression of cancer lesions according to three factors: lump size and spread within the breast (T classification), lymph node metastasis (N classification), and distant metastasis (M classification). Stage determination based on the UICC-TNM classification can be made in accordance with common knowledge of those skilled in the art.

Specifically, Stage 0 is defined as breast cancer that remains within mammary ducts; Stage I is defined as breast cancer with a tumor diameter of 2 cm or less without axillary lymph node metastasis or with micrometastasis of 0.2 mm or less; Stage II is defined as tumor diameter greater than 2 cm without axillary lymph node metastasis or tumor diameter of 5 cm or less with 3 or less axillary lymph node metastases; and Stage III is defined as 4-9 axillary lymph node metastases regardless of tumor diameter (including clinically evident parasternal lymph node metastases even in the absence of axillary lymph node metastases), or tumor diameter greater than 5 cm with 9 or fewer axillary lymph nodes, or tumor invasion of the chest wall, skin ulcer, skin satellite node, or skin edema regardless of tumor diameter, or inflammatory breast cancer, regardless of lymph node metastases. When there are 10 or more axillary lymph node metastases or axillary lymph node and parasternal lymph node metastases, or ipsilateral supraclavicular lymph node metastases, any tumor status is defined as Stage III. When distant metastasis is present, the tumor is defined as Stage IV. In the following Examples, all cases are shown in terms of the stage after postoperative pathological diagnosis (p-stage) is made.

Classification by hormone receptor expression status refers to the expression status of estrogen receptor (ER) and/or progesterone receptor (PgR), such as presence or absence of expression (positive or negative) or high or low expression. The expression status of ER and PgR may be the expression status of genes encoding these proteins, but preferably the expression status of these proteins. Classification by HER2 gene and/or protein expression status may be based on the presence or absence of the HER2 gene and/or protein (positive or negative) or high or low expression of the HER2 gene and/or protein. Methods for measuring the expression status of ER, PgR, and HER2 are known to those skilled in the art and are not limited, and examples of methods for detecting proteins include an immunological detection method such as immunohistochemical staining, and examples of methods for detecting nucleic acids include a nucleic acid amplification method using a primer or a hybridization method using a probe (such as FISH (Fluorescence In Situ Hybridization) method.

When ER, PgR, and HER2 are combined for classification, classification can be carried out into the following three groups: (1) hormone receptor positive/HER2 negative, in which ER and/or PgR are expressed and HER2 is not expressed; (2) HER2 positive, in which HER2 is expressed regardless of the presence or absence of the expression of ER and PgR; and (3) triple negative, in which neither ER, PgR, nor HER2 is expressed.

In one embodiment, the presence or absence or high or low expression of a CK2α protein or a fragment thereof in the nucleolus is used as a marker for predicting the prognosis of a cancer patient. The presence or absence or high or low expression thereof is described in detail below.

In one aspect, the present invention relates to a method for predicting the prognosis of a cancer patient. The present method comprises: a step of detecting a CK2α protein or a fragment thereof in a nucleolus in a cancer cell or a tissue obtained from a cancer patient; and a step of predicting a poor prognosis when a CK2α protein or a fragment thereof is detected and/or predicting a good prognosis when a CK2α protein or a fragment thereof is not detected. The detection step can be performed *in vitro.* In addition, a CK2α protein or a fragment thereof in a nucleolus can be detected using the above-described anti-CK2α antibody or an immunoresponsive fragment thereof.

In one aspect, the present invention relates to a method for predicting the prognosis of a cancer patient. The present method comprises a step of detecting a CK2α protein or a fragment thereof in a nucleolus in a cancer cell or a tissue obtained from a cancer patient; and a step of predicting a poor prognosis when a CK2α protein or a fragment thereof is detected in a nucleolus at a higher level compared with other cell fractions, and/or predicting a good prognosis when a CK2α protein or a fragment thereof is not detected in a nucleolus at a higher level compared with other cell fractions. In this connection, "other cell fractions" are not limited if the fractions are cell fractions other than the nucleolus, and can be, for example, cytoplasm or nucleoplasm (nucleosol). In addition, "when a CK2α protein or a fragment thereof is not detected in a nucleolus at a higher level compared with other cell fractions" comprises a case where a CK2α protein or a fragment thereof is detected in a nucleolus to the same extent as in other cell fractions (including a case where a CK2α protein or a fragment thereof is detected uniformly throughout the cell) and a case where a CK2α protein or a fragment thereof is detected in other cell fractions at a higher level than in the nucleolus. The detection step can be performed *in vitro.* In addition, a CK2α protein or a fragment thereof in a nucleolus can be detected using the above-described anti-CK2α antibody or an immunoresponsive fragment thereof.

Each step is described in detail below.

### (1) Detection step

The stage of cancer that a patient as a subject of the present invention suffers from is not limited. For example, in the case of breast cancer, the breast cancer which a patient as a subject of the present invention suffers from may be breast cancer of stage I-IV, such as stage I-III or stage III.

The cancer patient in the present invention is, for example, a mammal, preferably a primate, and more preferably a human.

Cancer cells or a tissue used in the present invention can be obtained from a cancer patient, for example, by biopsy or surgical resection, although not particularly limited thereto. The cells or tissue may be used directly for detection of a marker, or may be pretreated as appropriate for measurement. For example, paraffin-embedded sections may be prepared from patient-derived samples when the marker is detected by immunohistochemical staining. For example, when the biomarker is detected by Western blotting, a protein extract may be prepared by isolating a nucleus or a nucleolus from a patient-derived sample.

A marker to be detected in the present method may be any of a CK2α protein or a fragment thereof. The detection encompasses measurement of, *e.g*., the presence or absence of expression, the amount of expression, or the larger or smaller concentration of expression. As used herein, the term "detection" encompasses any of measurement, qualitative evaluation, quantification, and semi-quantification.

The method for detecting a CK2α protein or a fragment thereof may be any known protein detection method and is not particularly limited, and examples thereof include an immunological detection method.

The "immunological detection method" is a method for measuring the amount of a target molecule using an antibody or antibody fragment that specifically binds to the target molecule which is an antigen. Specifically, in the present step, the above-described CK2α protein or a fragment thereof can be used.

Examples of an immunological detection method include an immunohistochemical staining, an enzyme immunoassay measurement (including ELISA method and EIA method), Western blotting, radioimmunoassay (RIA), immunoprecipitation, chromatin immunoprecipitation (CHIP), or flow cytometry.

For the "immunohistochemical staining method," any known method can be used. For example, a patient-derived sample may be fixed in formalin, embedded in paraffin, sectioned into tissue pieces, and attached to a glass slide as a section sample. Immunohistochemical staining may be performed for a section sample using a primary antibody (specifically the above-described CK2α protein or a fragment thereof) that recognizes a CK2α protein or a fragment thereof and a labeled secondary antibody that recognizes the primary antibody, optionally after antigen retrieval by heat treatment.

In a method in which an expression site cannot be confirmed, such as Western blotting, the expression of a CK2α protein or a fragment thereof in a nucleolus can be confirmed by using a sample obtained by isolating the nucleolus in advance.

Each of the above-described measurement methods is a known technology in the field. Therefore, a specific measurement method can be performed in accordance with a known method. For example, a method described in Green, M.R. and Sambrook, J., 2012 (described above) can be referred to.

### (2) Prediction step

In the present step, the prognosis of a cancer patient is predicted based on a measurement result obtained in the above-described measurement step. In one embodiment, the present step comprises determining whether the cancer cell or the tissue is positive or negative for the marker from the results obtained in the above-described detection step. When the cancer cell or the tissue is negative for the marker, the prognosis for the cancer patient can be predicted as good. On the other hand, when the cancer cell or the tissue is positive for the marker, the prognosis for the cancer patient can be predicted to be poor.

When immunohistochemical staining is used, for example, a case in which one or a plurality of cells or cell clusters are stained can be determined as positive, and a case in which there is no number of stained tumor cells can be determined as negative. Alternatively, a case in which the number of stained tumor cells exceeds a certain proportion (for example, 10%, 15%, or 20%) of the total number of tumor cells may be determined as positive, and a case in which the number of stained tumor cells is not more than the above-described proportion of the total number of tumor cells may be determined as negative. In an immunohistochemical staining method, for example, a section can be classified into the following five stages: I, II, III, IV, and V.
I: there is staining of a whole cell, but nucleus staining is not clear
II: nucleus staining (+), with nucleus staining clearer than cytoplasm
III: nucleus staining (++), with nucleus staining at a higher level than II
IV: nucleus staining (+, ++), with nucleolus staining (+)
V: nucleus staining (-), with nucleolus staining (+)

In the above-described classification, IV and V can be determined to be positive for a CK2α protein or a fragment thereof in a nucleolus.

In one embodiment, the prediction step comprises determining whether the expression level of the marker in the cancer cells or the tissue obtained in the detection step is higher or lower (for example, than a certain threshold). When the expression level of a marker in cancer cells or a tissue is lower than a certain threshold (for example, statistically significantly lower), the prognosis of a cancer patient can be predicted to be good (for example, relative to a population having an expression level higher than a certain threshold). On the other hand, when the expression level of a biomarker in a cancer cell or a tissue is higher than a certain threshold (for example, statistically significantly higher), the prognosis of a cancer patient can be predicted to be poor (for example, relative to a population having an expression level lower than a certain threshold).

The certain threshold may be a control amount measured in a control sample (a control cell or a tissue, such as a control mammary cell or a mammary tissue). The control sample may be derived from a healthy individual (for example, a healthy human), a benign tumor of a mammary gland, or a breast cancer patient (for example, a Stage II breast cancer patient). In the present invention, "healthy individual" refers to a healthy individual of the same species as a subject individual who is not suffering from a cancer.

For example, the expression levels in these individuals or the median value, the average value, the upper level, the lower level, or a value within a certain range in a plurality of individuals can be used as a certain threshold. The threshold can be set as appropriate according to *e.g*., the precision of the prediction, and can be determined, for example, by ROC (receiver operating characteristic curve) analysis.

As used herein, "statistically significant" refers to a case in which the risk rate (significance level) of the obtained value is small, specifically p < 0.05 (less than 5%), p < 0.01 (less than 1%) or p < 0.001 (less than 0.1%). For the statistical test method, any known test method that can determine the presence or absence of significance may be used as appropriate, and is not particularly limited. For example, Student t-test, multiple comparison test, and log-rank test can be used.

As used herein, "poor prognosis" means that the clinical outcome is poor (unfavorable) (for example, after surgical resection) (for example, high recurrence risk or recurrence rate of a cancer such as a breast cancer, low recurrence-free survival rate, low disease (cancer)-specific survival rate, or low overall survival rate). When the prognosis is poor, the recurrence-free survival rate or disease-specific survival rate after 5 years may be 95% or less, 90% or less, 85% or less, 80% or less, 75% or less, or 70% or less. In the present invention, the survival rate means the cumulative survival rate.

As used herein, "good prognosis" means that the clinical outcome is good (favorable). When the prognosis is good, the recurrence-free survival rate or survival rate after 5 years from surgical resection of a cancer may be 90% or more, 95% or more, or 100%.

According to the present invention, the prognosis of a cancer patient can be predicted, and based on the results, a treatment strategy (for example, the type, the dosage, and the interval of administration of an anticancer drug) can be determined, or an interval for testing for cancer recurrence and metastasis can be determined.

When the present invention predicts that the prognosis of a cancer patient is poor, a drug therapy and/or radiation therapy can be carried out for the patient in order to prevent recurrence of the cancer or to improve the prognosis or to improve the survival rate. Therefore, the present invention also provides a method for preventing recurrence of a cancer or improving prognosis or improving survival rate, comprising administering at least one of a drug therapy and a radiation therapy to a cancer patient who is predicted to have a poor prognosis by the method of the present invention. Further, when the prognosis of a cancer patient is predicted to be poor, the frequency of testing may be increased in order to detect cancer recurrence at an earlier stage.

Examples of a drug comprise, but are not limited to, an anticancer agent such as doxorubicin, cyclophosphamide, 5-fluorouracil (5-FU), capecitabine, oxaliplatin, and irinotecan; a hormonal therapeutic agent such as an antiestrogen agent (for example, tamoxifen), an LH-RH agonist formulation (for example, leuplin), an aromatase inhibitor (for example, anastrozole), and a progesterone preparation; and an antibody drug such as a HER2 antibody (for example, trastuzumab). A drug can be used alone or in combination. A drug can be administered through a route such as injection, intravenous administration, or oral administration.

In one embodiment, the method described herein combine the presence or absence of detection of a CK2α protein or a fragment thereof with a factor such as classification by stage, tumor diameter, presence or absence of lymph node metastasis, or histological grade, for predicting the prognosis of a cancer patient.

In one embodiment, in the method described herein, cancer is breast cancer, and the presence or absence of detection of a CK2α protein or a fragment thereof is combined with at least one of classification by stage, classification by hormone receptor expression status, and classification by HER2 gene and/or protein expression status, for predicting the prognosis of a breast cancer patient. Classification by stage, hormone receptor expression status, and HER2 gene and/or protein expression status are described in the section (Use as a marker for predicting prognosis). In one embodiment, the method described herein predicts the prognosis of a breast cancer patient by combining the presence or absence of detection of a CK2α protein or a fragment thereof with another factor such as tumor diameter, presence or absence of lymph node metastasis, or histological grade, in addition to or separately from the above-described classification. Combination with another classification or factor can produce an effect of enabling better prediction of prognosis.

Hereinafter, the present invention will be described more specifically using Examples. However, the technical scope of the present invention is not limited to these Examples.

### Examples

### <Example 1: Production of anti-CK2α monoclonal antibody>

### (Purpose)

A monoclonal antibody (anti-CK2α monoclonal antibody) that can specifically detect a CK2α protein with high sensitivity is to be developed.

### (Method and result)

### (1) Immunization with antigen polypeptide

The production of an anti-CK2α monoclonal antibody was performed by entrusting Immuno-Biological Laboratories Co, Ltd with part of the production. Specifically, a human CK2α protein of SEQ ID NO: 2 to which a GST tag is bound was expressed in *E. coli* (DE3). Then, the GST tag was cleaved by thrombin treatment, and the resulting polypeptide was purified as an antigen. Then, Immuno-Biological Laboratories Co, Ltd was entrusted with the immunization of mice. After the immunization, blood was collected for testing, and the antibody titer was examined by ELISA.

Figure 1A shows the results of examining an antiserum obtained from a murine individual after immunization for the reactivity to a recombinant CK2α protein and a recombinant CK2α' protein by Western blotting. In the Western blotting, an anti-mouse IgG-HRP antibody (Abcam plc, #6789) was used as a secondary antibody, and detection was performed using a chemiluminescent detection reagent (Thermo Scientific, #32209). The antiserum obtained from the murine individual exhibited reactivity to both of the CK2α protein and the CK2α' protein, whose structure is similar to the structure of the CK2α protein. The results suggested that most of the anti-CK2α antibodies produced by mice immunized with a human CK2α protein exhibit cross-reactivity to a CK2α' protein.

### (2) Cell fusion and screening

Lymphocytes were isolated from the murine individual after immunization. The lymphocytes and myeloma cells were fused, and then, antibody production in the culture supernatant was subjected to screening using ELISA. Then, the hybridomas in faverable wells were subjected to cloning by limiting dilution method and screening by ELISA. As clones of hybridomas that did not react with the CK2α' protein, and exhibited selective reactivity to the CK2α protein, four clones 6A, 6B, 6C, and 7A were obtained.

### (3) Analysis of selected clones

Figure 1B shows the results of Western blotting for a recombinant CK2α protein and a recombinant CK2α' protein using the culture supernatants of clones 6A, 6B, 6C, and 7A. All of the clones 6A, 6B, 6C, and 7A reacted selectively with the CK2α protein, and exhibited no reactivity at all to the CK2α' protein.

### (4) Further cloning for clone 6A

Further cloning was performed for clone 6A by limiting dilution method, and clones 6A1, 6A2, and 6A3 were obtained. Figure 2 shows the results of detecting a CK2α protein in a cytoplasmic lysate of Flag-CK2α-expressing HEK293 cells, using culture supernatants of the clones 6A1, 6A2, and 6A3. Figure 2 also shows, as a control ("Control antibody" in Figure 2), the results of detection using a commercially available mouse anti-CK2α monoclonal antibody (ab70774, Abcam, UK). With the control antibody, a nonspecific band was detected at the side of higher molecular weight, in addition to the band of the CK2α protein. With the clones 6A1, 6A2, and 6A3, no such band was detected. The clones 6A1, 6A2, and 6A3 were demonstrated to be antibodies of extremely high specificity.

### (5) IgG purification from clone 6A3

The clone 6A3 was proliferated by serum-supplemented culture, and then further cultured in a serum-free media. Then, from the culture supernatant collected, IgG was purified using a protein A column. Hereinafter, the monoclonal antibody after purification is referred to as "mAb (6A3)".

### <Example 2: Western blotting and immunoprecipitation using anti-CK2α monoclonal antibody mAb (6A3)>

### (Purpose)

Western blotting and immunoprecipitation were performed using the anti-CK2α monoclonal antibody mAb (6A3) produced in Example 1. The performance of mAb (6A3) and a commercially available mouse anti-CK2α monoclonal antibody (ab70774, Abcam, UK; hereinafter referred to as a "control antibody") is to be compared.

### (Method and result)

### (1) Western blotting for cellular endogenous CK2α protein

Using mAb (6A3) at 0.1 µg/mL or 0.5 µg/mL, Western blotting was performed for a cytoplasmic lysate of HEK293 cells for detecting an endogenous CK2α protein.

The results are shown in Figure 3. Figure 3 also shows the results of detection using a mouse anti-CK2α monoclonal antibody (ab70774, Abcam, UK) at 0.5 µg/mL as a control ("Control antibody" in Figure 3). As shown in Figure 3B, mAb (6A3) at 0.5 µg/mL successfully detected an endogenous CK2α protein with sensitivity more than 3.5 times higher than the control antibody at the same concentration. In addition, mAb (6A3) at 0.1 µg/mL successfully detected an endogenous CK2α protein with sensitivity more than 2 times higher than the control antibody at the concentration of 0.5 µg/mL.

These results demonstrated that mAb (6A3) can detect an endogenous CK2α protein with remakably higher sensitivity than conventional antibodies.

### (2) Western blotting for Flag-CK2α protein

Using mAb (6A3) at 0.1 µg/mL and the control antibody at 0.5 µg/mL, Western blotting was performed for a cytoplasmic lysate of Flag-CK2α protein-expressing HEK293 cells.

The results are shown in Figure 4. With the control antibody, nonspecific bands were detected (two bands shown by the arrows in Figure 4, right) at the same position as for the Flag-CK2α protein also in the cells not expressing the Flag-CK2α protein (lanes of Flag-CK2α (-)). In contrast, in the detection by mAb (6A3), a band of the Flag-CK2α protein was detected only in the cells expressing the Flag-CK2α protein (lane of Flag-CK2α (+)), and nonspecific band was not detected at the same positions as that of the Flag-CK2α protein (the positions shown by the arrows in Figure 4, left) in the cells not expressing the Flag-CK2α protein (lane of Flag-CK2α (-)).

These results demonstrated that mAb (6A3) can detect the CK2α protein with higher specificity than conventional antibodies. In addition, the results demonstrated that mAb (6A3) can specifically detect both of cellular endogenous CK2α and CK2α expressed as an exogenous gene, in a cell lysate.

### (3) Western blotting for immunoprecipitates

A lysate was prepared from each of the cytoplasm and nucleus of HEK293 cells. From each of the cytoplasm lysate and the nucleus lysate, immunoprecipitation was performed using 1 µg of mAb (6A3) or a mouse anti-CK2α monoclonal antibody (ab70774, Abcam, UK). Specifically, HEK293 cells cultured were washed with PBS, and then collected from the plate. The cytoplasmic fractions and the nucleus fractions were partially purified in accordance with a common method. An anti-CK2α antibody was added, and mixing was performed mildly at 4°C for two hours to form a CK2α immune complex. Then, the immunoprecipitates obtained by centrifugation were treated with SDS, and then electrophoresed. For Western blotting, mAb (6A3) or a mouse anti-CK2α monoclonal antibody (ab70774, Abcam, UK) was used at 4 µg/mL.

The results of Western blotting are shown in Figure 5. In addition, Figure 6 shows the results as a sum of quantified staining intensities, for each of the bands of CK2α in the gels shown on the left in Figure 5 and the bands of CK2α in the gels shown on the right in Figure 5. When mAb (6A3) or the control antibody was used as an antibody for immunoprecipitation, a large difference was not detected in the amount of the immunoprecipitated endogenous CK2α protein. On the other hand, when mAb (6A3) or the control antibody was used as an antibody for Western blotting, mAb (6A3) was able to detect the endogenous CK2α protein with higher sensitivity.

### (4) Further Western blotting for immunoprecipitates

A cytoplasmic lysate of Flag-CK2α-expressing RPE cells was prepared. From this lysate, immunoprecipitation was performed using 1 µg of mAb (6A3) or a mouse anti-CK2α monoclonal antibody (ab70774, Abcam plc, UK). For the resulting immunoprecipitates, Western blotting was performed using mAb (6A3) at 0.1 µg/mL or an anti-Flag antibody (#1805, Sigma) at 2 µg/mL.

The results of Western blotting are shown in Figure 7. In addition, Figure 8 shows the results of quantification of staining intensities of the two bands shown by the arrow in Figure 7. When mAb (6A3) or the control antibody was used as an antibody for immunoprecipitation, the amount of immunoprecipitated Flag-CK2α protein was larger with mAb (6A3) (comparison between the two bands shown by the arrow in Figure 7).

### <Example 3: Immunohistochemistry in a breast cancer tissue using anti-CK2α monoclonal antibody mAb (6A3)>

### (Purpose)

A breast cancer tissue is subjected to immunostaining using the anti-CK2α monoclonal antibody mAb (6A3). The CK2α protein localized to the nucleolus in cancer cells is to be detected using mAb (6A3) and a commercially available mouse anti-CK2α monoclonal antibody (ab70774, Abcam, UK; hereinafter referred to as a "control antibody"), and the performance of the two antibodies is to be compared.

### (Method and result)

Formalin-fixed, paraffin-embedded specimens of cancer invasion sites (lesion sites) in breast cancer tissues resected from patients with primary breast cancer (invasive breast ductal carcinoma) in first breast cancer extirpation between 2007 and 2014 at Hoshi General Hospital were used. Tumor stage was p Stage IIB according to the TNM classification of malignant tumors (UICC International Code, L.H. Sobin, M.K. Gospodarowicz and Ch. Wittekind, TNM Classification of Malignant Tumours, 7th edition). This study was approved by the review boards of Hoshi General Hospital and Fukushima Medical University.

A formalin block was sectioned at 4 µm thickness and mounted on a glass plate. Tissue Tech Prisma 6120 (Sakura Finetech Japan Co., Ltd.) was used in deparaffinization and rehydration in accordance with common methods, and an antigen was retrieved by autoclaving at 105°C for 10 min in 10 mM sodium bicarbonate buffer (pH 8.0). The section was subjected to blocking with goat serum diluted 200-fold in 10 mM phosphate-buffered saline (PBS) containing 1% bovine serum albumin (BSA) for 30 minutes at room temperature. After the section was washed with PBS, reaction with the anti-CK2α monoclonal antibody mAb (6A3) or a mouse anti-CK2α monoclonal antibody (ab70774, Abcam, UK) was performed overnight at 4°C in PBS containing BSA and 0.05% Tween^{®} 20. After 16 hours, the section was incubated with Biotin-conjugated anti-mouse IgG (BA-9200, Vector Laboratories, US) for 30 minutes at room temperature and then with Vectastain^{™} Elite ABC HRP kit (PK-6102, Vector Laboratories, US) for 30 minutes with an avidin-horse radish peroxidase complex, followed by visualization of the anti-CK2α antibody with Diaminobenzidine (DOJINDO, Japan) under acidic conditions.

In this regard, the anti-CK2α monoclonal antibody mAb (6A3) was used at 1,000-fold dilution (0.1 µg/mL). Further, the mouse anti-CK2α monoclonal antibody (ab70774, Abcam, UK) was used at the dilution of 1,000-fold (2 µg/mL).

Figure 9 shows the results of staining in a cancer invasion site (lesion site) of invasive breast ductal carcinoma. In the staining using mAb (6A3) at 0.1 µg/mL, the nucleolus portion which is an intranuclear structure was strongly stained, and on the other hand, the staining of the background was low (Figure 9A). As a result, the nucleolus in which the CK2α protein is localized in cancer cells was clearly distinguishable. On the other hand, in the staining using the control antibody at 2 µg/mL, the staining of the nucleolus portion was weak and not distinctly different from the background (Figure 9B).

### <Example 4: Determination of CDR sequences of the anti-CK2α monoclonal antibody mAb (6A3)>

### (Purpose)

The variable region sequences and CDR sequences of the heavy chain and light chain of the anti-CK2α monoclonal antibody mAb (6A3) are to be determined.

### (Method and result)

With respect to the clone 6A3, the determination of the amino acid sequence of the antibody was consigned to Fasmac Co., Ltd. The results of determining the variable region sequence and CDR sequence of each of the heavy chain and the light chain are shown below. It is noted that CDRs were identified in accordance with the antibody numbering system of Kabat. In addition, it was also found that mAb (6A3) is IgG2b.

### [Table 2]

**Table 2: Amino acid sequence of anti-CK2α monoclonal antibody mAb (6A3)**

| Region | Amino acid sequence | SEQ ID NO |
|---|---|---|
| Heavy chain variable region | MEWSWVFLFLLSVIAGVQSQVQLQQSGAELVRPGASV TLSCKASGYKFTDYQMHWVKQTPVHGLEWIGVIDPGT GGTAYNQKFKGKAILTADKSSSTAYMELRSLTSEDSA VYYCTGFVWGTGTTVTVSSAKTTPPSVYPLAPGCGDT TGSSVTLGCLVKGYFPESVT | 11 |
| Light chain variable region | MDMRAPAQIFGFLLLLFPGSRCDIQMTQSPSSLSASL GERVSLTCRASQDIGSSLNWLQQEPDGTIKRLIYATS SLDSGVPKRFSGSRSGSDYSLTISSLESEDFVDYYCL QYAIFPYTFGGGTKLEIKRADAAPTVSIFPPSSEQLT SGGASVVCFLN | 12 |
| Heavy chain CDR1 | DYQMH | 5 |
| Heavy chain CDR2 | VIDPGTGGTAYNQKFKG | 6 |
| Heavy chain CDR3 | FV | 7 |
| Light chain CDR1 | RASQDIGSSLN | 8 |
| Light chain CDR2 | ATSSLDS | 9 |
| Light chain CDR3 | LQYAIFPYT | 10 |

### <Example 5: Production of further anti-CK2α monoclonal antibodies>

### (Purpose)

Monoclonal antibodies (anti-CK2α monoclonal antibodies) that can specifically detect a CK2α protein with high sensitivity are to be further developed. Furthermore, the variable region sequence and CDR sequence of the heavy chain and light chain are to be determined for each clone.

### (Method and result)

Mice were immunized with a human CK2α protein as an antigen in accordance with the method described in "(1) Immunization with antigen polypeptide" in Example 1. Furthermore, in accordance with the methods described in "(2) Cell fusion and screening", "(3) Analysis of selected clones", and "(4) Further cloning for clone 6A" in Example 1, lymphocytes were isolated from the murine individuals after immunization. The lymphocytes and the myeloma cells were fused to produce antibody-producing hybridomas, and five clones: clones 10B2, 15C1, 16C2, 19C2, and 21B1 were obtained. Furthermore, in accordance with the method described in "(5) IgG purification from clone 6A3" in Example 1, the culture supernatant of each clone was purified to obtain "mAb (10B2)", "mAb (15C1)", "mAb (16C2)", "mAb (19C2)", and "mAb (21B1)" as purified monoclonal antibodies.

Next, in accordance with the same method as in Example 4, the variable region sequence and CDR sequence of the heavy chain and light chain were determined for each of the obtained anti-CK2α monoclonal antibodies. The results obtained by determining the variable region sequence and CDR sequence of each of the heavy chain and the light chain are shown below. It is noted that the CDRs were identified in accordance with the antibody numbering system of Kabat. In addition, it was found that, among the obtained anti-CK2α monoclonal antibodies, "mAb (6A3)", "mAb (10B2)", "mAb (15C1)", and "mAb (16C2)" are all IgG2b, and "mAb (19C2)" and "mAb (21B1)" are IgG1.

### [Table 3]

**Table 3: Amino acid sequence of anti-CK2α monoclonal antibody mAb (1082)**

| Region | Amino acid sequence | SEQ ID NO |
|---|---|---|
| Heavy chain variable region | MEWSWVFLFLLSVIAGVQSQVQLQQSGAE LVRPGASVTLSCKASGYTFTDYQMHWVKQ TPVHGLEWIGVIDPGTGGTAYNQKFKGKA ILTADKSSNTAYMELRSLTSEDSAVYYCT GFVWGTGSPVTVSSAKTTPPS | 15 |
| Light chain variable region | MDMRAPAQIFGFLLLLFPGSRCDIQMTQS PSSLSASLGERVSLTCRASQDIGNNLNWL QQEPDGTIKRLIYATSSLDSGVPKRFTGS RSGSDYSLTISSLESEDFVDYYCLQYAIF PYTFGGGTKLEIKRADAAP | 16 |
| Heavy chain CDR1 | DYQMH | 17 |
| Heavy chain CDR2 | VIDPGTGGTAYNQKFKG | 18 |
| Heavy chain CDR3 | FV | 19 |
| Light chain CDR1 | RASQDIGNNLN | 20 |
| Light chain CDR2 | ATSSLDS | 21 |
| Light chain CDR3 | LQYAIFPYT | 22 |

### [Table 4]

**Table 4: Amino acid sequence of anti-CK2α monoclonal antibody mAb (15C1)**

| Region | Amino acid sequence | SEQ ID NO |
|---|---|---|
| Heavy chain variable region | MEWSWVFLFLLSVIAGVQSQVQLQQSGAE LVRPGASVTLSCKASGYTFTDFQMHWVKQ TPVHGLEWIGVIDPGTGGTAYNQKFKDKA ILTADKSSNTAYMELRSLTSEDSAVYYCT GFVWGTGTTVTVSSAKTTPPS | 23 |
| Light chain variable region | MDMRAPAQIFGFLLLLFPGSRCDIQMTQS PSSLSASLGERVSLTCRASQDIGNNLNWL QQEPDGTIKRLIYATSSLDSGVPKRFTGS RSGSDYSLTISSLESEDFVDYYCLQYAIF PYTFGGGTKLEIKRADAAP | 24 |
| Heavy chain CDR1 | DFQMH | 25 |
| Heavy chain CDR2 | VIDPGTGGTAYNQKFKD | 26 |
| Heavy chain CDR3 | FV | 27 |
| Light chain CDR1 | RASQDIGNNLN | 28 |
| Light chain CDR2 | ATSSLDS | 29 |
| Light chain CDR3 | LQYAIFPYT | 30 |

### [Table 5]

**Table 5: Amino acid sequence of anti-CK2α monoclonal antibody mAb (16C2)**

| Region | Amino acid sequence | SEQ ID NO |
|---|---|---|
| Heavy chain variable region | MEWSWVFLFLLSVIAGVQSQVQLQQSGAE LVRPGASVTLSCKASGYKFTDYQMHWVKQ TPVHGLEWIGVLDPGTGGTAYNQKFRGKA ILTADKSSSTAYMELRSLTSEDSAVYYCT GFVWGTGTTVTVSSAKTTPPS | 31 |
| Light chain variable region | MDMRAPAQIFGFLLLLFPGSRCDIQMTQS PSSLSASLGERVSLTCRASQDIGSSLNWL QQEPDGTIKRLIYATSSLDSGVPKRFSGS RSGSDYSLTISSLESEDFVDYYCLQYAIF PYTFGGGTKLEIKRADAAP | 32 |
| Heavy chain CDR1 | DYQMH | 33 |
| Heavy chain CDR2 | VLDPGTGGTAYNQKFRG | 34 |
| Heavy chain CDR3 | FV | 35 |
| Light chain CDR1 | RASQDIGSSLN | 36 |
| Light chain CDR2 | ATSSLDS | 37 |
| Light chain CDR3 | LQYAIFPYT | 38 |

### [Table 6]

**Table 6: Amino acid sequence of anti-CK2α monoclonal antibody mAb (19C2)**

| Region | Amino acid sequence | SEQ ID NO |
|---|---|---|
| Heavy chain variable region | MSSPQTLNTLTLTMGWSWIFLFLLSGTAG VHSEVQLQQSGPVLVKPGASVKMSCKASG YTFTDYYMNWVKQSHGKSLEWIGVIDPNN GGTSYNQKFKGKATLTVDKSSSTAYMELN SLTSEDSAVYYCPRMGGNFYFDYWGQGTT LTVSSAKTTPPSVYPLAPGSAAQTNSMVT LGCLVKGYFPEPVT | 39 |
| Light chain variable region | MRCLAEFLGLLVLWIPGAIGDIVMTQAAP SVPVTPGESVSISCRSSKSLLHSNGNTYL YWFLQRPGQSPQLLIYRMSNLASGVPDRF SGSGSGTAFTLRISRVEAEDVGVYYCMQH LEFPYTFGGGTKLEIKRADAAPTVSIFPP SSEQLTSGGASVVCFLN | 40 |
| Heavy chain CDR1 | DYYMN | 41 |
| Heavy chain CDR2 | VIDPNNGGTSYNQKFKG | 42 |
| Heavy chain CDR3 | MGGNFYFDY | 43 |
| Light chain CDR1 | RSSKSLLHSNGNTYLY | 44 |
| Light chain CDR2 | RMSNLAS | 45 |
| Light chain CDR3 | MQHLEFPYT | 46 |

### [Table 7]

**Table 7: Amino acid sequence of anti-CK2α monoclonal anitbody mAb (21B1)**

| Region | Amino acid sequence | SEQ ID NO |
|---|---|---|
| Heavy chain variable region | MSSSQSLNTLTLTMEWIWIFLFILSGTAGVQ SQVQLQQSGAELTRPGASVKLSCKASGYTFT SFGISWVKQTTGQGLEWIGEIYPRSGNTYYN EKFKGKATLTADKSSSTAFMELRSLTTEDSA VYFCEGDWYFDVWGTGTTVTVSSAKTTPPSV YPLAPGSAAQTNSMVTLGCLVKGYFPEPVT | 47 |
| Light chain variable region | MMSPAQFLFLSVLWIRETNGDVVMTQTPLTL SVTIGQPASISCKSSQSLLDSDGKTYLNWLL QRPGQSPKRLIYLVSKLDSGVPDRFTGSGSG TDFTLKISRVEAEDLGVYYCWQGTHFPHTFG GGTKLEMKRADAAPTVSIFPPSSEQLTSGGA SVVCFLN | 48 |
| Heavy chain CDR1 | SFGIS | 49 |
| Heavy chain CDR2 | EIYPRSGNTYYNEKFKG | 50 |
| Heavy chain CDR3 | DWYFDV | 51 |
| Light chain CDR1 | KSSQSLLDSDGKTYLN | 52 |
| Light chain CDR2 | LVSKLDS | 53 |
| Light chain CDR3 | WQGTHFPHT | 54 |

### <Example 6: Western blotting and chromatin immunoprecipitation using anti-CK2α monoclonal antibodies>

### (Purpose)

Western blotting and immunoprecipitation are carried out for comparing the performance of the anti-CK2α monoclonal antibody mAb (6A3) produced in Example 1; the anti-CK2α monoclonal antibodies, mAb (10B2), mAb (15C1), mAb (16C2), and mAb (19C2), produced in Example 5; and a commercially available mouse anti-CK2α monoclonal antibody (ab70774, Abcam, UK; referred to as a "control antibody").

### (Method and result)

### (1) Western blotting for the immunoprecipitates from cell lysate

A lysate was prepared from a breast cancer cell line MCF-7 (JCRB cell bank, #JCRB0134) and Flag-CK2α protein-expressing HEK293 cells. The MCF-7 cell line or Flag-CK2α-protein-expressing HEK293 cells were cultured, and washed with PBS, and then collected from the plate. A soluble fraction was partially purified from the cell lysate in accordance with common methods, and the anti-CK2α antibody (1 µg of six antibodies of the present invention or the control antibody) was added. Then, mixing was performed mildly at 4°C for two hours, and then, Protein G-agarose beads were added, and a CK2α immune complex was formed. Then, the immunoprecipitates obtained by centrifugation were treated with SDS, and then electrophoresed. In Western blotting, the antibody of the present invention or the control antibody was used at 0.1 µg/mL.

Figure 10 shows the results of Western blotting after the immunoprecipitation, for the MCF-7 cell line or Flag-CK2α protein-expressing HEK293 cells. The Western blotting performed with the control antibody detected, in the MCF-7 cell line, a band of the endogenous CK2α and in addition, a nonspecific band equal to or stronger than the band, and also detected, in the Flag-CK2α protein-expressing HEK293 cells, bands of the endogenous CK2α and Flag-CK2α, and in addition, a nonspecific band equal to or stronger than the bands. In contrast, mAb (6A3), mAb (10B2), mAb (15C1), mAb (16C2), and mAb (19C2) of the present invention did not detect this nonspecific band, demonstrating a higher specificity to CK2α.

### (2) Chromatin immunoprecipitation

The CK2α protein is recruited in a wide range of sites on the human genome including the HMGB2 (High Mobility Group Box 2) gene locus in the nucleus (the present inventors, unpublished). Based on this, the performance of the antibody of the present invention is investigated by performing chromatin immunoprecipitation for the HMGB2 gene locus.

Specifically, a lysate was produced in accordance with common methods of chromatin immunoprecipitation from human retinal pigment epithelium cells (human RPE cells) and human RPE cells (RPE-ko) in which the CK2 gene is knocked out using a CRISPR-Cas 9 method, and a chromatin fraction was prepared. An anti-CK2α antibody (1 µg of mAb (6A3), mAb (10B2), mAb (15C1), or mAb (16C2)) was added to this chromatin fraction, and mixing was performed mildly at 4°C using a rotator overnight, and then Protein G-agarose beads were added before carrying out centrifuge for obtaining a fraction of a chromatin immune complex. Using the nucleic acids contained in the resulting fraction as a template and a primer pair of SEQ ID NOs: 65 and 66, the base sequence of the human HMGB2 gene locus was amplified by PCR. The amplification conditions for the PCR (temperature and time) were as follows: at 95°C for 3 seconds and at 60°C for 20 seconds (in one cycle); and the cycle was repeated 40 times. The DNA amplification amount corresponding to the HMGB2 was measured by Applied Biosystems Step One (Life Technologies Corp.) using a KAPA SYBR Fast qPCR kit (KAPA Biosystems, Inc.).

The results of chromatin immunoprecipitation are shown in Figure 11. The fraction immunoprecipitated with any one of the antibodies, mAb (6A3), mAb (10B2), mAb (15C1), and mAb (16C2), exhibited a larger amplification amount of the target sequence corresponding to the HMGB2 gene locus than the sample to which specific antibody was not added ("Control" in Figure 11). This result demonstrated that the chromatin corresponding to the HMGB2 gene locus was effectively concentrated in the fraction immunoprecipitated with the above-described antibodies.

### (3) Western blotting for recombinant CK2α

Using mAb (6A3), mAb (10B2), mAb (15C1), mAb (16C2), mAb (19C2), and mAb (21B1) of the present invention, a purified product of recombinant CK2α protein was detected by Western blotting. The recombinant CK2α protein obtained by purifying the human-derived CK2α protein expressed by introducing the pGEX2T-CK2α gene in *E. coli* was used. The recombinant CK2α protein in an amount of 0.05 µg, 0.1 µg, or 0.2 µg was treated with SDS, electrophoresed, and was subjected to detection with six antibodies of the present invention at 0.1 µg/mL.

The results of Western blotting are shown in Figure 12. It was demonstrated that all of mAb (6A3), mAb (10B2), mAb (15C1), mAb (16C2), mAb (19C2), and mAb (21B1) can detect the recombinant CK2α protein.

### <Example 7: Immunohistochemistry in breast cancer tissue using anti-CK2α monoclonal antibody mAb (21B1)>

### (Purpose)

The CK2α protein localized to the nucleolus in cancer cells are detected by immunostaining in a breast cancer tissue using the anti-CK2α monoclonal antibody mAb (21B1) produced in Example 5 and a control antibody.

### (Method and result)

Formalin-fixed, paraffin-embedded specimens of a cancer invasion site (lesion site) in a breast cancer tissue were immunostained with the anti-CK2α monoclonal antibody mAb (21B 1) in accordance with the method described in Example 3. The anti-CK2α monoclonal antibody mAb (21B1) was used at the dilution of 1,000-fold (0.1 µg/mL). In addition, the mouse anti-CK2α monoclonal antibody (ab70774, Abcam, UK) was used at the dilution of 1,000-fold (2 µg/mL).

Figure 9 shows the results of staining in the cancer invasion site (lesion site) of invasive breast ductal carcinoma. In the staining using mAb (21B1) at 0.1 µg/mL, the nucleolus portion which is an intranuclear structure was strongly stained, whereas the staining of the background was low (Figure 13A). As a result, the nucleolus in which the CK2α protein was localized was clearly distinguishable in the cancer cell. On the other hand, in the staining using the control antibody at 2 µg/mL, the staining of the nucleolus portion was weak, showing no clear difference from the background (Figure 13B).

### <Example 8: Evaluation of prognosis of lung adenocarcinoma patients after surgical resection>

### (Purpose)

The expression and localization of the CK2α protein are evaluated in a lung adenocarcinoma tissue resected from lung adenocarcinoma patients using the monoclonal anti-CK2α antibody mAb (6A3) of the present invention, and the prognosis of the lung adenocarcinoma patients after the surgical resection is evaluated.

### (Method)

Formalin-fixed, paraffin-embedded specimens of lung adenocarcinoma tissue resected from 120 patients with primary lung adenocarcinoma who were subjected to radical resection between 2007 and 2014 in the Department of Chest Surgery at Fukushima Medical University were used. Tumor stage was determined according to the TNM classification of malignant tumors (UICC International Code, L.H. Sobin, M.K. Gospodarowicz and Ch. Wittekind, TNM Classification of Malignant Tumours, 8th edition). This study was approved by the review boards of Fukushima Medical University.

A formalin block was sectioned at 4 µm thickness and mounted on a glass plate. Tissue Tech Prisma 6120 (Sakura Finetech Japan Co., Ltd.) was used in deparaffinization and rehydration in accordance with common methods, and an antigen was retrieved by autoclaving at 105°C for 10 min in 10 mM sodium bicarbonate buffer (pH 8.0). The section was subjected to blocking with goat serum diluted 200-fold in 10 mM phosphate-buffered saline (PBS) containing 1% bovine serum albumin (BSA) for 30 minutes at room temperature. After the section was washed with PBS, reaction with the anti-CK2α monoclonal antibody mAb (6A3, 0.1 µg/mL) was performed overnight at 4°C in PBS containing BSA and 0.05% Tween^{®} 20. After 16 hours, the section was incubated with Biotin-conjugated anti-mouse IgG (BA-9200, Vector Laboratories, US) for 30 minutes at room temperature and then with Vectastain^{™} Elite ABC HRP kit (PK-6102, Vector Laboratories, US) for 30 minutes with an avidin-horse radish peroxidase complex, followed by visualization of the anti-CK2α antibody with Diaminobenzidine (DOJINDO, Japan) under acidic conditions. Serial sections were counterstained with hematoxylin.

An immunohistochemical slide with a CK2α antibody was evaluated by two independent pathologists who did not know the patient information at 5 stages of I, II, III, IV, and V according to the following criteria.
I: whole cell is stained, with nucleus staining unclear
II: nucleus staining (+), with nucleus staining clearer than cytoplasm
III: nucleus staining (++), with nucleus staining at a higher level than II
IV: nucleus staining (+, ++), with nucleolus staining (+)
V: nucleus staining (-), with nucleolus staining (+)

With 118 primary lung adenocarcinoma patients at stages I-III excluding stage IV, the survival curves of CK2α nucleolus staining positive (IV + V) group and negative (I + II + III) group were analyzed by the Kaplan-Meier method. Specifically, the recurrence-free survival was analyzed. Significant differences between the two survival curves for CK2α nucleolus staining positive (IV+V) and negative (I+II+III) were tested by log-rank test, and hazard ratios and 95% confidence intervals were calculated. All statistical analyses were performed using JMP Pro version 14.2.0.

Furthermore, the prognosis of 118 primary lung adenocarcinoma patients at stages I-III excluding stage IV was evaluated. Patient clinical information was obtained retrospectively by reviewing medical records. Prognostic events were recurrence, death due to lung adenocarcinoma, and all-cause death, and the relationship with the evaluation of CK2α staining was examined.

### (Results)

The results of recurrence-free survival rate are shown in Figure 14. Lung adenocarcinoma patients being positive with CK2α nucleolus staining showed a significantly lower recurrence-free survival rate compared to patients being negative with nucleolus staining, indicating that the recurrence risk of CK2α nucleolus staining positive cases is significantly higher than that of negative cases (N = 118, P = 0.0031, log-rank test).

In addition, a univariate analysis and a multivariate analysis were performed for 120 primary lung adenocarcinoma patients. The univariate analysis focused on each variable, and the intensities of correlation with and influence on the variable of interest, *i.e.*, the postoperative length of recurrence-free survival of those who had first lung adenocarcinoma, were evaluated. In the multivariate analysis, four variables for which the P value indicated less than 0.05 in the univariate analysis were selected, and it was analyzed whether the variables mutually influenced the recurrence-free survival period, or whether the variables are independent variables to specify the variable of interest.

The results of calculation of the hazard ratio (shown as "HR" in the figure, indicating relative likelihood of recurrence) and the 95% confidence interval thereof in the univariate analysis and the multivariate analysis are shown in Figure 15A and Figure 15B. Furthermore, for analyzing the effect of predicting the length of recurrence-free survival in the multivariate analysis, the results of comparing the degree of contribution to the variable of interest using JMP Pro version 14 Partition Tree are shown in Figure 15C. This result showed statistical significance of the independent variable, demonstrating that positive CK2α nucleolus staining is an indicator strongly related to future recurrence. Additionally, in the multivariate analysis, the Wald value, which is a test statistic showing statistical significance, was largest for the index of positive CK2α nucleolus staining.

Figure 16 shows the results of analyzing variables that predict the length of time before/until recurrence, using two recurrence prediction models.

Figure 16A shows the results of the recurrence prediction model 1 based on six variables. With respect to the median value of 1397 days regarding the length of time before/until recurrence, the top variable that contributed most thereto was the case in which metastasis to the surrounding lymphatic glands was present at the operation, which represented 717 days, and in contrast, the case of no metastasis to the surrounding lymphatic glands represented 1496 days. On the other hand, the case of positive CK2α nucleolus staining represented 1326 days, in which the case of (positive CK2α nucleolus staining and) lymphatic vessel invasion represented 745 days.

Figure 16B shows the results of the recurrence prediction model 2 based on seven variables including age in addition to six variables in the model 1. The top variable was the case in which metastasis to the surrounding lymphatic glands was present at the operation (representing 717 days, with respect to the median value of 1397 days regarding the length of time before/until recurrence). With respect of the case of no metastasis to the surrounding lymphatic glands (1496 days), the age of 80 or more represented 845 days. On the other hand, with respect to the age of less than 80 (1566 days), the case of positive CK2α nucleolus staining represented 1370 days, in which the case with (positive CK2α nucleolus staining and) the presence of lymphatic vessel invasion represented 745 days.

Figure 17 shows primary lung adenocarcinoma patients at Stages I to III, classified by stage and according to the evaluation of CK2α staining, and the distinction of the presence or absence of recurrence is shown. It was statistically suggested that, particularly in clinical cancer stages, positive CK2α nucleolus staining is effective as an index indicative of the possibility of future recurrence, even in the cases classified into early stages according to the size of the first cancer and according to low or no metastasis to the surrounding lymphatic glands (Figure 17).

All publications, patents and patent applications cited herein are directly incorporated herein by reference.

## Claims

1. An anti-CK2α antibody or a fragment thereof, comprising:
(1) a heavy chain variable region comprising:
CDR1 consisting of the amino acid sequence of SEQ ID NO: 5,
CDR2 consisting of the amino acid sequence of SEQ ID NO: 6, and
CDR3 consisting of an amino acid sequence FV, and
a light chain variable region comprising:
CDR1 consisting of the amino acid sequence of SEQ ID NO: 8,
CDR2 consisting of the amino acid sequence of SEQ ID NO: 9, and
CDR3 consisting of the amino acid sequence of SEQ ID NO: 10;
(2) a heavy chain variable region comprising:
CDR1 consisting of the amino acid sequence of SEQ ID NO: 17,
CDR2 consisting of the amino acid sequence of SEQ ID NO: 18, and
CDR3 consisting of an amino acid sequence FV, and
a light chain variable region comprising:
CDR1 consisting of the amino acid sequence of SEQ ID NO: 20,
CDR2 consisting of the amino acid sequence of SEQ ID NO: 21, and
CDR3 consisting of the amino acid sequence of SEQ ID NO: 22;
(3) a heavy chain variable region comprising:
CDR1 consisting of the amino acid sequence of SEQ ID NO: 25,
CDR2 consisting of the amino acid sequence of SEQ ID NO: 26, and
CDR3 consisting of an amino acid sequence FV, and
a light chain variable region comprising:
CDR1 consisting of the amino acid sequence of SEQ ID NO: 28,
CDR2 consisting of the amino acid sequence of SEQ ID NO: 29, and
CDR3 consisting of the amino acid sequence of SEQ ID NO: 30;
(4) a heavy chain variable region comprising:
CDR1 consisting of the amino acid sequence of SEQ ID NO: 33,
CDR2 consisting of the amino acid sequence of SEQ ID NO: 34, and
CDR3 consisting of an amino acid sequence FV, and
a light chain variable region comprising:
CDR1 consisting of the amino acid sequence of SEQ ID NO: 36,
CDR2 consisting of the amino acid sequence of SEQ ID NO: 37, and
CDR3 consisting of the amino acid sequence of SEQ ID NO: 38;
(5) a heavy chain variable region comprising:
CDR1 consisting of the amino acid sequence of SEQ ID NO: 41,
CDR2 consisting of the amino acid sequence of SEQ ID NO: 42, and
CDR3 consisting of the amino acid sequence of SEQ ID NO: 43, and
a light chain variable region comprising:
CDR1 consisting of the amino acid sequence of SEQ ID NO: 44,
CDR2 consisting of the amino acid sequence of SEQ ID NO: 45, and
CDR3 consisting of the amino acid sequence of SEQ ID NO: 46; or
(6) a heavy chain variable region comprising:
CDR1 consisting of the amino acid sequence of SEQ ID NO: 49,
CDR2 consisting of the amino acid sequence of SEQ ID NO: 50, and
CDR3 consisting of the amino acid sequence of SEQ ID NO: 51, and
a light chain variable region comprising:
CDR1 consisting of the amino acid sequence of SEQ ID NO: 52,
CDR2 consisting of the amino acid sequence of SEQ ID NO: 53, and
CDR3 consisting of the amino acid sequence of SEQ ID NO: 54.

2. The anti-CK2α antibody or a fragment thereof according to claim 1, comprising:
(a) a heavy chain variable region consisting of the amino acid sequence of SEQ ID NO: 11, and a light chain variable region consisting of the amino acid sequence of SEQ ID NO: 12;
(b) a heavy chain variable region consisting of the amino acid sequence of SEQ ID NO: 15, and a light chain variable region consisting of the amino acid sequence of SEQ ID NO: 16;
(c) a heavy chain variable region consisting of the amino acid sequence of SEQ ID NO: 23, and a light chain variable region consisting of the amino acid sequence of SEQ ID NO: 24;
(d) a heavy chain variable region consisting of the amino acid sequence of SEQ ID NO: 31, and a light chain variable region consisting of the amino acid sequence of SEQ ID NO: 32;
(e) a heavy chain variable region consisting of the amino acid sequence of SEQ ID NO: 39, and a light chain variable region consisting of the amino acid sequence of SEQ ID NO: 40; or
(f) a heavy chain variable region consisting of the amino acid sequence of SEQ ID NO: 47, and a light chain variable region consisting of the amino acid sequence of SEQ ID NO: 48.

3. A kit for predicting the prognosis of a cancer patient, comprising the anti-CK2α antibody or a fragment thereof according to claim 1 or 2.

4. The kit according to claim 3, wherein the prognosis comprises recurrence risk.

5. The kit according to claim 3 or 4, wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.

6. A method for predicting the prognosis of a cancer patient, the method comprising:
a step of detecting a CK2α protein or a fragment thereof in a nucleolus in a cancer cell or a tissue obtained from a cancer patient; and
a step of predicting a poor prognosis when a CK2α protein or a fragment thereof is detected in a nucleolus at a higher level compared with other cell fractions, and/or predicting a good prognosis when a CK2α protein or a fragment thereof is not detected in a nucleolus at a higher level compared with other cell fractions,
wherein the CK2α protein or a fragment thereof is detected with the anti-CK2α antibody or a fragment thereof according to claim 1 or 2.

7. The method according to claim 6, wherein the prognosis comprises recurrence risk.

8. The method according to claim 6 or 7, wherein said cancer is selected from the group consisting of breast cancer, uterine cancer, esophageal cancer, gastric cancer, biliary tract cancer, pancreatic cancer, liver cancer, renal cancer, colorectal cancer, bladder cancer, lung cancer, thyroid cancer, and glioma.

9. The method according to claim 8, wherein said cancer is breast cancer, and whether a CK2α protein or a fragment thereof is detected or not is combined with at least one of classification by stage, classification by hormone receptor expression status, and classification by HER2 gene and/or protein expression status to predict the prognosis of a breast cancer patient.
